# EUROPEAN PATENT APPLICATION

(11) **EP 4 455 295 A1**
(43) Date of publication of application: **30.10.2024**
(21) Application number: 23382397.0
(22) Date of filing: 27.04.2023
(51) Int. Cl.: C12N 15/86, C12N 15/87

(54) **GENE THERAPY FOR THE TREATMENT OF GLUTARIC ACIDURIA TYPE I**

(71) Applicant: Fundació de Recerca Clínic Barcelona-Institut d'Investigacions Biomèdiques August Pi I Sunyer, 08036 Barcelona (ES); Hospital Clínic de Barcelona, 08036 Barcelona (ES); Consorcio Centro de Investigación Biomédica en Red, 28029 Madrid (ES)
(72) Inventor: FILLAT FONTS, Cristina, 08036 Barcelona (ES); GEA SORLI, Sabrina, 08036 Barcelona (ES); MATEU BOSH, Anna, 08036 Barcelona (ES); RIBES RUBIÓ, Antonia, 08036 Barcelona (ES); GARCIA VILLORIA, Judit, 08036 Barcelona (ES); SEGUR BAILACH, Eulàlia, 08036 Barcelona (ES); TORT ESCALÉ, Frederic, 08028 Barcelona (ES)
(74) Representative: Hoffmann Eitle

(57) **Abstract**

The present invention relates to gene therapy for the treatment of Glutaric Aciduria type I (GA-1), wherein the treatment comprises the administration of a nucleic acid comprising an expression cassette comprising the nucleotide sequence of a chimeric promoter and the nucleotide sequence encoding for the human glutaryl-CoA dehydrogenase enzyme (GCDH). Virions, cells and pharmaceutical composition comprising said nucleic acid are also provided.

## Description

### TECHNICAL FIELD

The present invention relates to the field of gene therapy, particularly in the treatment of Glutaric aciduria type I. Specifically, the present invention relates to a vector for gene therapy capable of treating Glutaric aciduria type I.

### BACKGROUND ART

Glutaric aciduria type I (GA-I, MIM 231670) is an inborn error of metabolism that was first described in 1975. It is a disease of low prevalence (1/70.000 to 1/100,000 newborns), whose inheritance is autosomal recessive. This disease is due to a deficiency of the enzyme glutaryl-CoA dehydrogenase (GCDH, EC 1.3.99.7), responsible for the dehydrogenation and decarboxylation of glutaryl-CoA in the catabolic pathway of lysine, hydroxylysine and tryptophan. The human GCDH gene is located on chromosome 19p13.2, expands 7Kb and consists of 11 exons and 10 introns. In general, GA-I is a heterogeneous disease from the genetic point of view, with more than 200 mutations identified. GCDH deficiency leads to an increase of glutaric acid (GA), 3-hydroxyglutaric acid (3-OHGA) and glutarylcarnitine (C5DC), and to a depletion of carnitine in body fluids. Alterations of these metabolites in plasma and urine are used for diagnosis. Most of the untreated individuals develop a complex movement disorder between the age of 3-36 months, due to bilateral striatal damage precipitated by catabolic events such as febrile illness, vomiting/diarrhea caused by infections or surgical procedures. After an episode of this type, dystonia, choreoathetosis movements and spasticity are observed, with severe neurological damage typical of an acute encephalopathic crisis. In a relevant number of patients, symptoms develop insidiously since birth, with macrocephaly or slightly delayed motor development, without an apparent clinical crisis, often resulting in a less severe disease. Life expectancy is very variable, there are patients with severe neurological damage who have reached adulthood, but approximately half of them, die during the first decade of life in the course of an acute episode.

A genetic mouse model of GA I has been developed to better understand the disease pathology. Mice knock out with complete loss of GCDH (*Gcdh* -/-) are viable with normal appearance but present a biochemical profile very similar to patients with GA I, with high levels of GA and 3-OHGA in urine and brain and high levels of GC in serum. Exposure of *Gcdh* -/- mice to elevated dietary protein of lysine aggravates disease presentation with tissue and serum accumulation of metabolites and noticeable signs of striatal neurodegeneration. The model shows age-dependent susceptibility to brain injury, similar to the human condition, with strong lethality when animals are fed with high lysine at weanling. Animals that survive have severe neuropathological alterations, with neuronal loss, vacuolation and in some cases with intraventricular hemorrhages. Alterations in the glutamatergic system have been observed, suggesting greater susceptibility to excitotoxic neuronal damage.

Studies in these mice have been key for the development of therapeutic strategies. The exposure of mice to a low lysine diet and high glucose was found to reduce the accumulation of neurotoxic metabolites in the brain (Sauer SW, et al. 2011. Therapeutic modulation of cerebral L-lysine metabolism in a mouse model for glutaric aciduria type I. Brain 134: 157-170), which is why most patients currently receive this type of diet in combination with an oral supplement of L-carnitine. This strategy allows to considerably reduce the frequency of acute encephalopathic episodes and therefore mortality (Kolker S, et al. (2007). Guideline for the diagnosis and management of glutaryl-CoA dehydrogenase deficiency (glutaric aciduria type I). J Inherit Metab Dis 30: 5-22). However, despite the efforts made for early detection, by including this disease in the newborn screening programs and the dietary treatment, some patients still develop encephalopathic crisis (Boy et al. (2020) Impact of newborn screening and quality of therapy on the neurological outcome in glutaric aciduria type 1: a metaanalysis).

Thus, there is an unmet need for an effective treatment for this disease. Gene therapy has been proven to be efficient in genetic diseases, and gene replacement strategies based on viral vectors have been proven to be effective in a variety of recessive genetic disorders. The authors of the present invention have explored the feasibility of a gene therapy strategy for glutaric aciduria type I based on the administration of an adeno-associated virus (AAV9) that transports the GCDH gene.

### BRIEF DESCRIPTION OF DRAWINGS

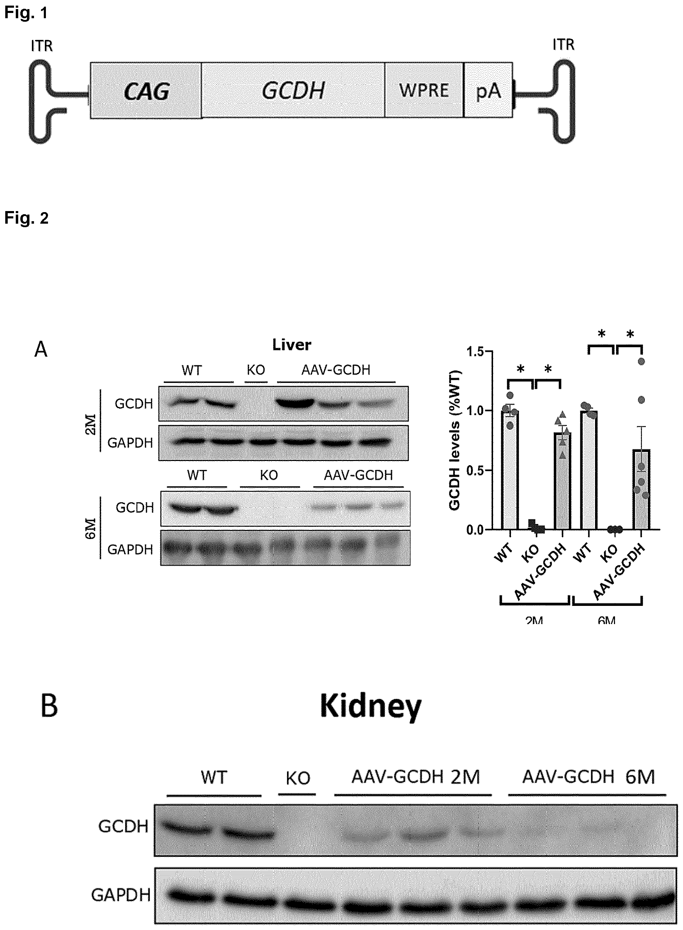

**Fig. 1****: Schematic representation of the expression cassette.**

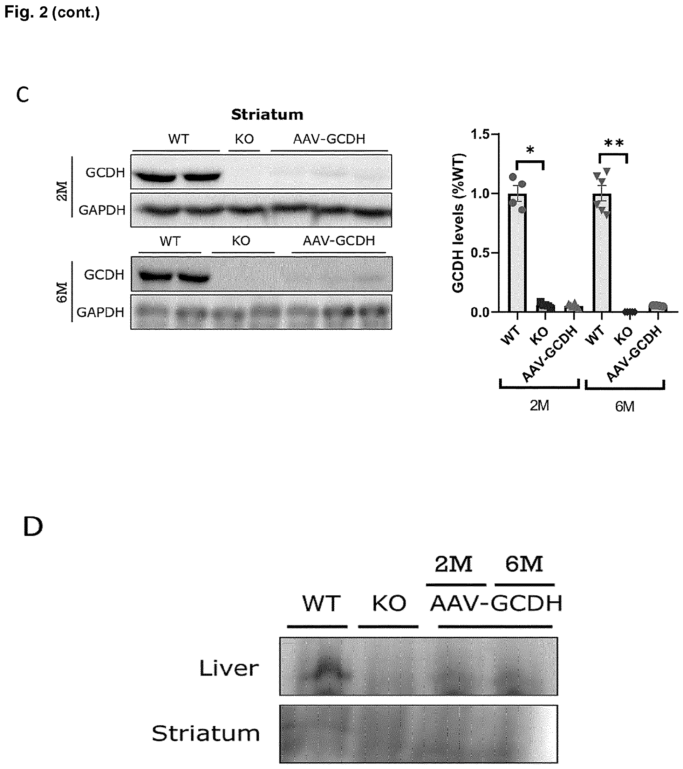

**Fig. 2****: Intravascular administration of AAV-GCDH in KO-adult mice results in long-term GCDH expression in liver but not in the striatum.** Western blot analysis of GCDH in liver (A), kidney (B) and striatum (C) lysates at 1 month and 5 months post-treatment. Quantification of GCDH from different individuals (n=4-6). (D) GCDH enzyme activity in liver and striatum at 1 month and 5 months post AAV-GCDH administration. Data are expressed as the means ± SEM. Significance was assessed using a two-tailed Mann-Whitney test. **P* < 0.05, ***P* < 0.01, ****P <* 0.001.

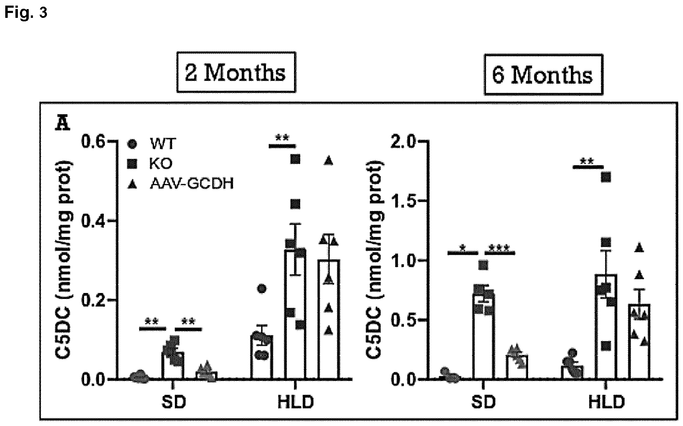

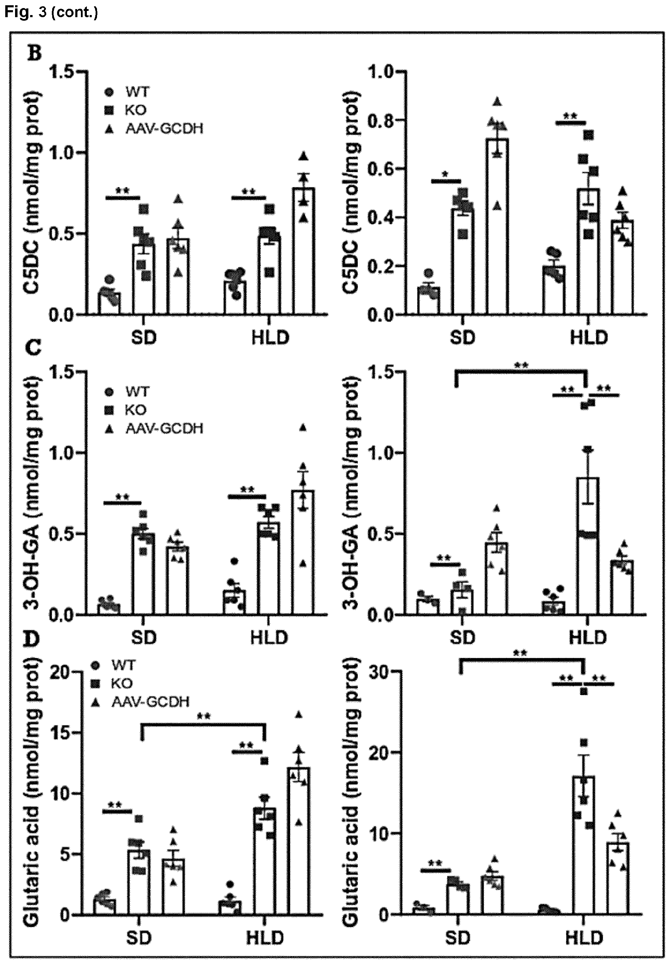

**Fig. 3****: Intravascular administration of AAV-GCDH in KO-young adult mice ameliorates HLD-induced metabolite accumulation in the striatum at 5-months post-treatment.** C5DC, GA and 3-OH GA were measured in WT, KO and AAV-GCDH treated mice one moth and five months post-treatment. C5DC accumulation in the liver (A) and the striatum (B). (C) GA accumulation in the striatum. (D) 3-OH GA accumulation in the striatum.

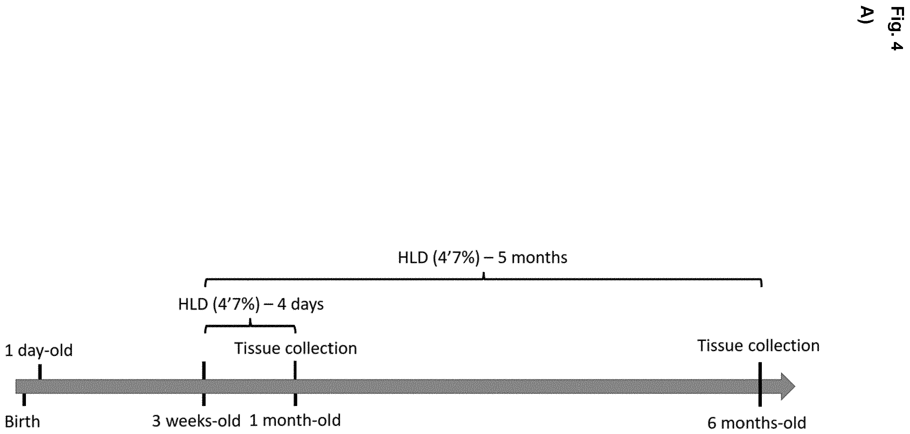

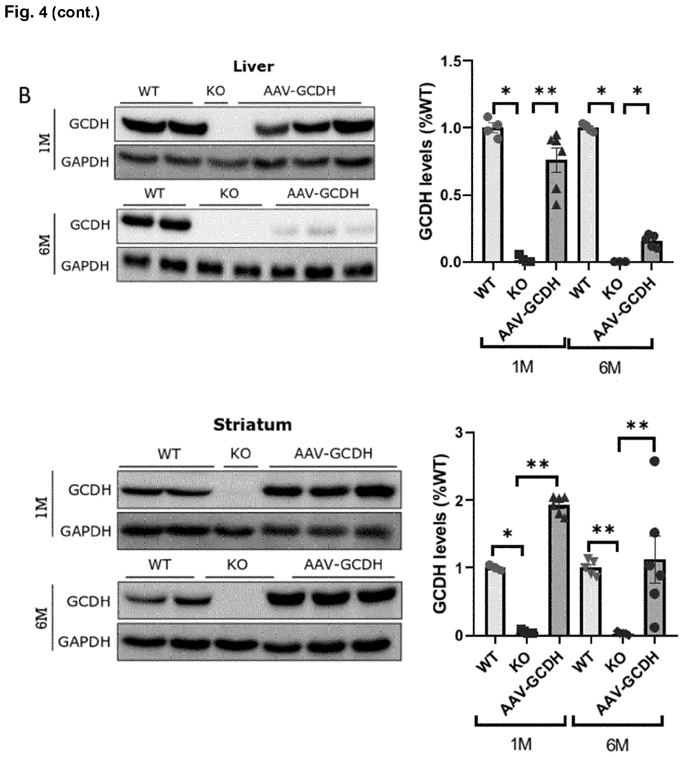

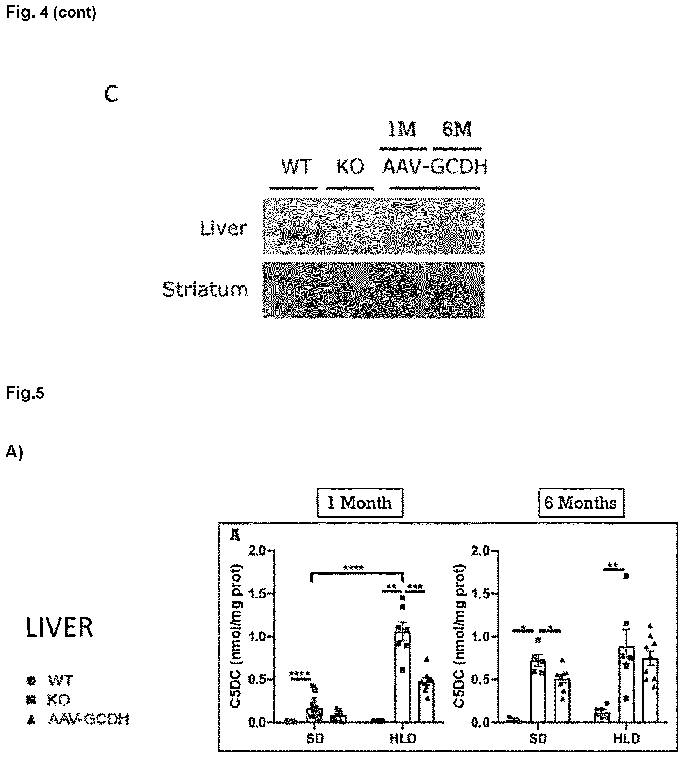

**Fig. 4****: Intravascular administration of AAV-GCDH in KO-neonatal mice displays normal levels of GDH and restores enzyme activity in liver and striatum.** (A). Scheme of AAV-GCDH application, HLD exposure and time-points of the readout analysis. AAV-GCDH were injected in the temporal vein at a dose (1×10¹¹ vg/mouse) at P1, at weanling were exposed to SD or to HLD for 4 days or 5 months. Readouts were performed at 1 month and 6 months post-viral injection. (B) Western blot analysis of GCDH in liver (upper panel) and striatum (lower panel) lysates at 1 month and 6 months post-treatment. Quantification of GCDH from different individuals (n=4-6). (C) GCDH enzyme activity in liver and striatum at 1 month and 6 months post AAV-GCDH administration. Data are expressed as the means ± SEM. Significance was assessed using a two-tailed Mann-Whitney test. **P* < 0.05, ***P* < 0.01, ****P* < 0.001.

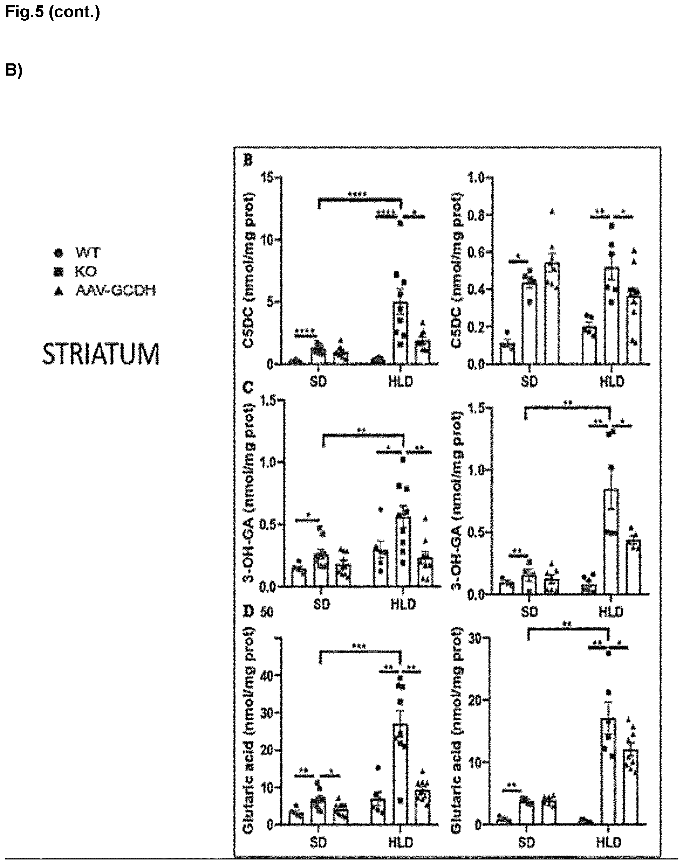

**Fig. 5****: Intravascular administration of AAV-GCDH in KO-neonatal mice prevents from HLD-induced metabolite accumulation in the striatum at 1-month and 6-months post-treatment.** C5DC, GA and 3-OH GA were measured in WT, KO and AAV-GCDH treated mice at the time points and conditions indicated in Figure 4A. C5DC accumulation in the liver (A) and the striatum (B). (C) GA accumulation in the striatum. (D) 3-OH GA accumulation in the striatum.

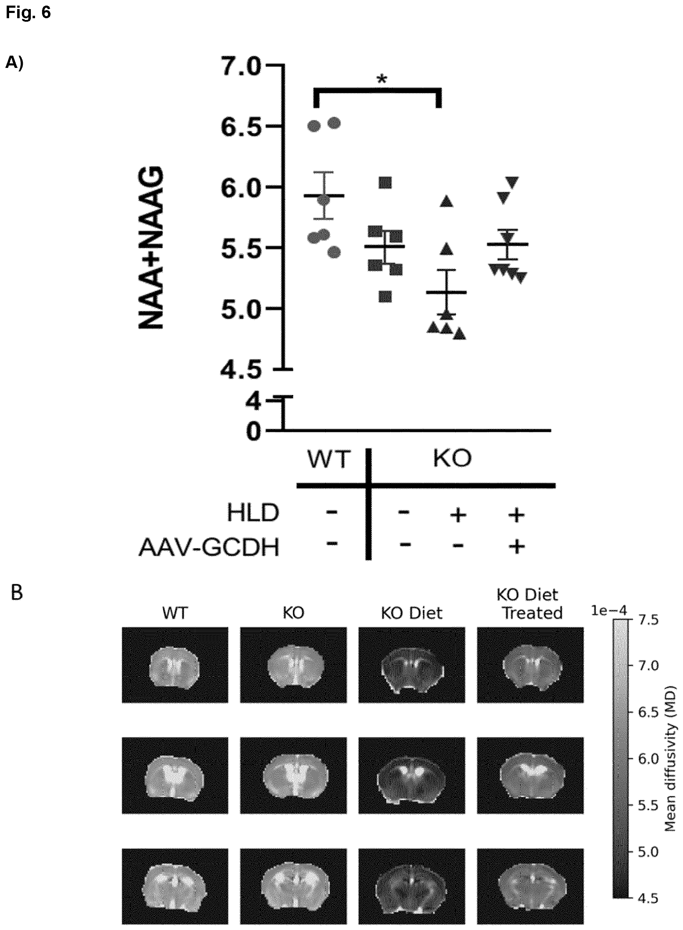

**Fig. 6****: MRI imaging.** Animals at on month age from WT, KO exposed to standard or HLD diet for 4 days and treated or not with AAV-GCDH mice were used for the MRI studies. (A). Proton MR spectroscopy imaging was performed in mice striatum. Calculation of N-acetyl- aspartate (NAA)+N-acetyl-aspartyl Glutamate (NAAG). (B) Brain images of Mean Diffusivity.

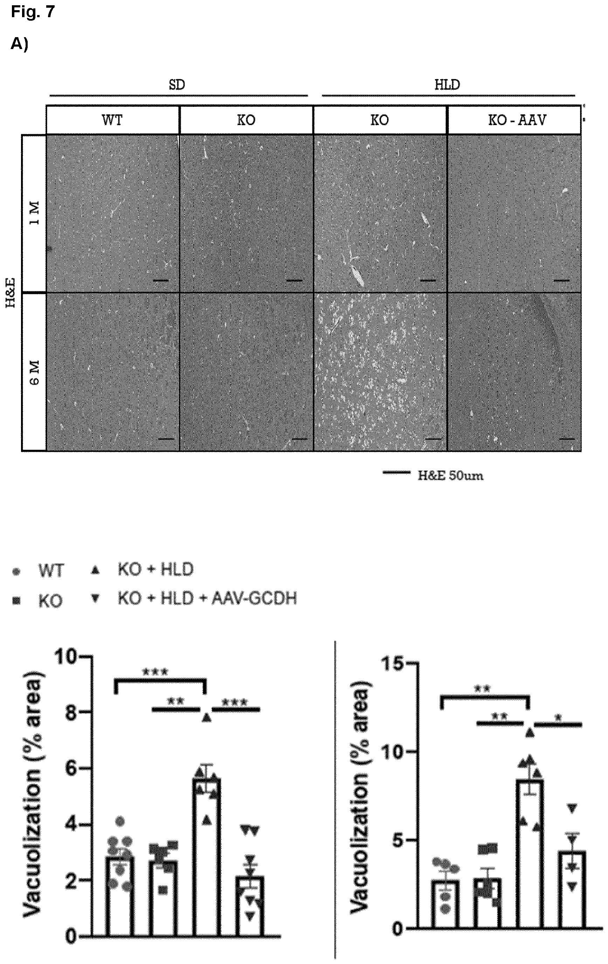

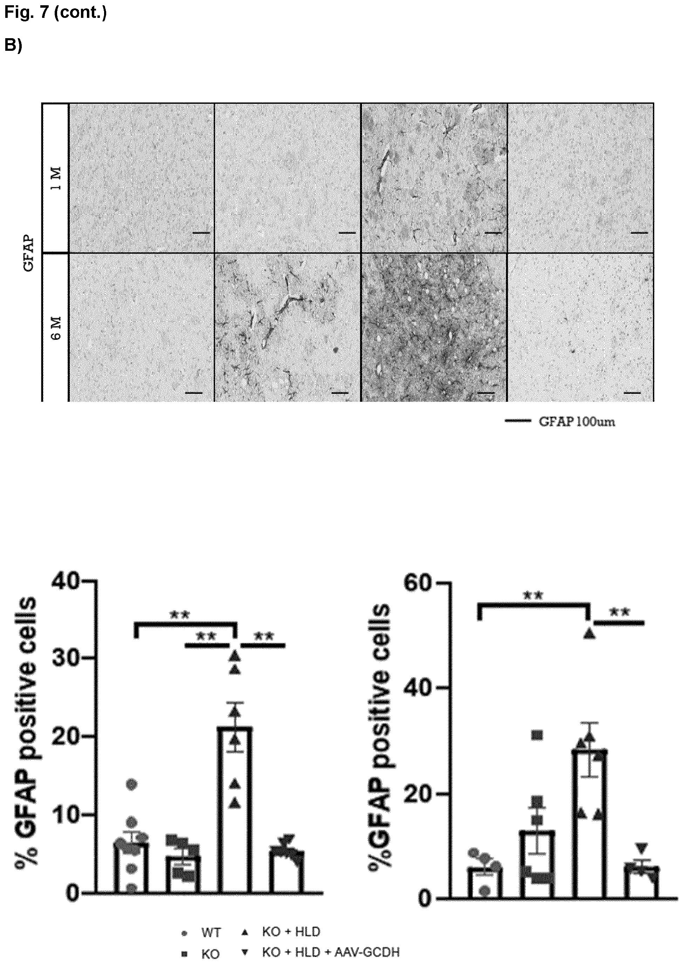

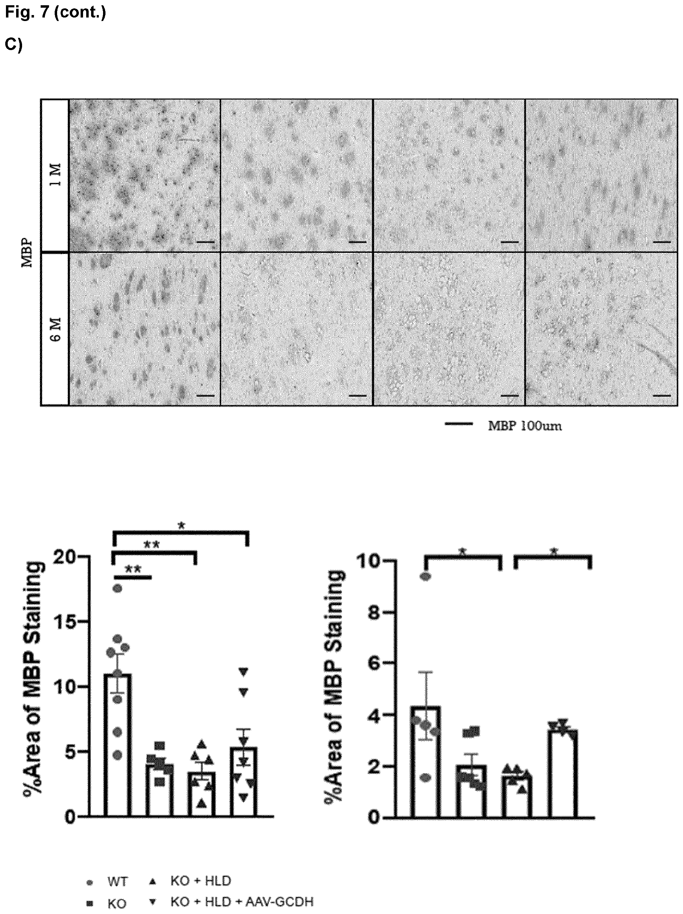

**Fig. 7****: Neonatal AAV-GCDH ameliorates striatal injury.** Immunohistochemical analysis of striatum alterations. A) Hematoxilin & Eosin staining showing progressive vacuolation in HLD mice, that appear as white holes. B) GFAP glial marker and C) MBP myelin marker. Quantification was performed on histology images with 1 section/animal digitalized with a ScanScope slide scanner and analyzed with QuPath 0.3.0 software. Results are presented as % area staining positive.

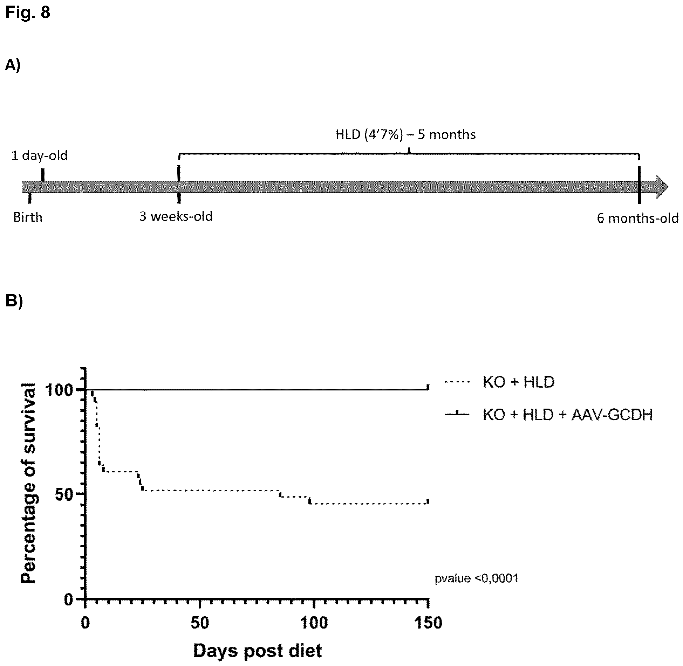

**Fig. 8****: Neonatal AAV-GCDH administration to HLD-KO mice results in normal lifespan**. A) Treatment scheme. B) Follow-up of 150 days mice survival in KO Gcdh -/- mice treated (n=25) or not (n=33) with AAV-GCDH and exposed to HLD at weanling.

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

It is noted that the term "about", as used herein, refers to +/- 30%, preferably +/- 20%, preferably +/- 15%, more preferably +/- 10%, of the indicated referred value.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an embodiment of the present invention may be substituted with the term "consisting of", though less preferred.

When used herein "consisting of" excludes any element, step, or ingredient not specified in the claim element. A protein or nucleotide that "consists essentially of" a protein or nucleotide is a protein or nucleotide that has considerably the same amino acid or nucleotide sequence as the specified protein or nucleotide.

"Percent (%) amino acid sequence identity" with respect to proteins or polypeptides described herein is defined as the percentage of amino acid residues in a candidate sequence that are identical with the amino acid residues in the reference sequence (i.e., the protein or polypeptide from which it is derived), after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for example, using publicly available computer software such as BLAST. Those skilled in the art can determine appropriate parameters for measuring alignment, including any algorithms needed to achieve maximum alignment over the full-length of the sequences being compared.

Preferably, the "percentage of identity" as used herein is decided in the context of a local alignment, i.e., it is based on the alignment of regions of local similarity between nucleobase sequences, contrary to a global alignment, which aims to align two sequences across their entire span. Thus, in the context of the present invention, percentage identity is calculated preferably only based on the local alignment comparison algorithm.

The term "complementary" and "complementarity" are interchangeable and refer to the ability of polynucleotides to form base pairs with one another. Base pairs are typically formed by hydrogen bonds between nucleotide units in antiparallel polynucleotide strands or regions. Complementary polynucleotide strands or regions can base pair in the Watson-Crick manner (e.g., A to T, A to U, C to G). 100% (or total) complementary refers to the situation in which each nucleotide unit of one polynucleotide strand or region can hydrogen bond with each nucleotide unit of a second polynucleotide strand or region. Less than perfect (or partial) complementarity refers to the situation in which some, but not all, nucleotide units of two strands or two regions can hydrogen bond with each other and can be expressed as a percentage. Please note that the term "complementary" as used in the present invention also encompasses the term "substantially complementary". A region is "substantially complementary" to a target region when the percentage of complementarity between both regions is of at least 60%, at least 70%, at least 80%, at least 85%, at least 90%, at least 93%, at least 95%, at least 96%, at least 97%, at least 98%, at least 99% or at least 100%. A region is "substantially complementary" to another region if they hybridize under low stringency conditions, preferably medium stringency conditions, most preferably high stringency conditions.

The term "expression" as used herein is defined as the transcription and/or translation of a particular nucleotide sequence driven by its promoter. A "coding region" or "coding sequence" is a portion of polynucleotide which consists of codons translatable into amino acids. The term "expression cassette" is used herein to refer to a polynucleotide sequence that comprises a coding sequence and one or more regulatory sequences, such as promoters and/or enhancers, that are operably linked to the coding sequence and control or affect or drive its expression.

"Encoding" refers to the inherent property of a nucleic acid to serve as a template, whether directly (i.e. a sense strand) or indirectly (i.e. an antisense strand) for synthesis of peptide, polypeptides, proteins, or other nucleic acids (i.e. rRNA, tRNA, microRNA). A nucleic acid can "encode" whether it is the sense strand, antisense strand, or a double-stranded segment thereof. The sense strand directly encodes the rRNA, tRNA, microRNA, or mRNA. The mRNA then serves as the template for translation of a peptide, polypeptide, or protein. The anti-sense strand is generally considered to be the reverse complementary sequence and is sometimes called a "noncoding" strand in the art (although for present purposes "non-coding" is a misnomer because the non-coding strand still "encodes" the genetic information by perpetuating it during semiconservative replication by acting as a template for the polymerization of a new, sense strand). By perpetuating the genetic information, the antisense strand is still encoding the genetic information for, for example, a protein. Accordingly, "a nucleic acid encoding X", includes sense and antisense sequences or strands whether X is a peptide, a polypeptide, or a protein or X is a sequence that encodes a rRNA, tRNA, microRNA, antisenseRNA, etc.

Further to which, "nucleic acid encoding X," includes RNA, DNA, and combinations thereof, since nucleic acids are synthesized from transcription, reverse-transcription, and replication, as naturally occurring processes and man-made processes (recombinant biology, molecular biology, etc). Accordingly, a recited nucleic acid sequence contemplates and supports the complementary version thereof, the reverse complementary version thereof, and double- stranded versions thereof.

The term "downstream" refers to a nucleotide sequence that is located 3' to a reference nucleotide sequence. In certain embodiments, downstream nucleotide sequences relate to sequences that follow the starting point of transcription. The term "upstream" refers to a nucleotide sequence that is located 5' to a reference nucleotide sequence. In certain embodiments, upstream nucleotide sequences relate to sequences that are located on the 5' side of a coding region or starting point of transcription

As used herein, the term "recombinant" refers a piece of DNA, protein, virus, etc. that has been created by combining two or more fragments from different sources. For instance, a recombinant AAV genome refers to a genome formed by combining at least two different fragments from different genomes.

The term "operably linked" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects or control the transcription or expression of the coding sequence.

The term "promoter" as used herein is defined as a DNA sequence recognized by the synthetic machinery of the cell, or introduced synthetic machinery, required to initiate the specific transcription of a coding polynucleotide sequence. In some instances, this sequence may be the core promoter and in other instances, this sequence may also include, or be an enhancer alone and/or other regulatory elements which are required for expression of the gene product. In certain instances the promoter may comprise enhancer elements, exons, and introns from one or a variety of viruses and animals, and thereby the term "promoter" shall be understood to not be limited to being a non-expressed sequence, nor exclude a non-expressed sequence that is between expressed sequences (introns), nor be limited to exclude an enhancer alone so long as the combination of sequences used to construct the promoter are capable of initiating and/or controlling the specific transcription of a coding polynucleotide sequence. The term "chimeric promoter" refers to a promoter that is formed by the combination of two or more regulatory elements, such as an enhancer and a promoter.

As used herein, the term "enhancer" refers to a nucleic acid sequence that when bound by an activator protein, activates or increases transcription of a gene or genes. Enhancer sequences can be up stream (i.e., 5') or downstream (i.e., 3 ') relative to the genes they regulate.

As used herein, the term "posttranscriptional response element" refers to a nucleic acid sequence that, when transcribed, adopts a tertiary structure that enhances expression of a gene.

A "target gene" refers to a nucleic acid encoding a target protein to be expressed within a target cell upon entry of the vector carrying the target gene into the cell. The target gene includes naturally occurring polymorphisms (i.e. variants) and man-made modifications to the wild-type gene so long as the target protein is still expressed. An example of such man-made modifications includes codon-optimization. Preferably, the target gene is the gene encoding for the human GCDH.

A "target protein" refers to a man-made or naturally occurring protein of interest to be introduced by vector into a host cell. One some embodiments, the target protein, as encoded in the genome of the host cell, is not functional because of a polymorphism in the gene sequence resulting in some mistranscription, missense, or mistranslation of the gene whereby reduced or no target protein or inoperable target protein is produced (i.e. a polymorphism results in an early stop codon) or an attenuation in the activity of the target protein, as encoded by and expressed from the genome of a subject. Preferably, the target protein is the human GCDH.

An "intron" is a region that resides within a gene but does not remain in the final mature mRNA molecule following transcription of that gene and does not code for amino acids that make up the protein encoded by that gene. Most protein-coding genes in the human genome consist of exons and introns. By "chimeric intron" is referred herein to an artificial intron to enhance mRNA processing and increase expression levels of the downstream ORF, which in this case is the human GCDH gene.

A "vector" is an entity (such as a nucleic acid or a virion) capable of delivering a target gene to the interior of a cell, and includes not only the expression-region (i.e. a promoter and a nucleic acid encoding a protein or even a nucleic acid), but also some cis-acting genetic component. The cis-acting genetic component provides for packaging within a virion, expression in a cell, replication in a cell, or a combination thereof. By way of example, a plasmid can comprise an origin of replication (e.g.. ori from cytomegalovirus) which allows for the replication of the target gene within a cell, and such a plasmid is thereby a vector. A viral genetic code may provide a nucleic acid sequence or protein encoded therein that allows for insertion of the gene of interest into the host genome, thereby providing for the replication of the target gene during the replication of, and within, the host cell's genome.

A "virion" is an entire virus particle comprising or consisting of an outer protein shell called a capsid and an inner core of nucleic acid (either ribonucleic or deoxyribonucleic acid). The core confers infectivity, and the capsid provides specificity to the virus.

The terms "treatment" and "therapy", as used in the present application, refer to a set of hygienic, pharmacological, surgical and/or physical means used with the intent to cure and/or alleviate a disease and/or symptoms with the goal of remediating the health problem. The terms "treatment" and "therapy" include preventive and curative methods, since both are directed to the maintenance and/or reestablishment of the health of an individual or animal. Regardless of the origin of the symptoms, disease and disability, the administration of a suitable medicament to alleviate and/or cure a health problem should be interpreted as a form of treatment or therapy within the context of this application. Preferably, the term "treatment" refers to the application or administration of a pharmaceutical composition to a subject who has a disease or disorder associated with low level of GCDH expression, with the purpose to cure, heal, alleviate, relieve, alter, remedy, ameliorate, improve, or affect the disorder, the symptom of the disease, or the predisposition toward a disease.

The term "therapeutically effective amount" refers to an amount of matter which has a therapeutic effect, and which is able to treat GA-I.

The terms "individual", "patient" or "subject" are used interchangeably in the present application and are not meant to be limiting in any way. The "individual", "patient" or "subject" can be of any age, sex and physical condition.

### DESCRIPTION OF THE EMBODIMENTS

Glutaric aciduria type I (GA-I) is a rare metabolic inherited disorder caused by the deficiency of glutaryl-CoA dehydrogenase (GCDH) leading to the accumulation of neurotoxic metabolites, resulting with a complex movement disorder and premature death. Currently, early diagnosis, through newborn screening and start of lysine restriction diet allow better prognosis and longer survival. However, these strategies do not reliably prevent the manifestation of severe movement disorder and untimely death in all screened individuals, encouraging the development of more effective therapies. A study by Guo et at. 2022 (Treatment of glutaric aciduria type I (GA-I) via intracerebroventricular delivery of GCDH https://doi.org/10.1016/j.fmre.2022.08.013 ) described the use of an AAV vector encoding for the GCDH. However, the AAV vector described in this study presents two mayor disadvantages. First, as shown in Fig. 3 of this study, only intracerebroventricular route showed the desired 100% efficacy, while 1×10¹¹ vg administered via retroorbital (i.e., systemic route) did not achieve a 100% of survival in mice. This lack of complete survival upon systemic administration may be associated to the fact that systemic injection only delivered AAV to the heart and liver (see Fig. S3b of Gue et al. 2022), while intracerebroventricular route delivered the AAV not only in the CNS but also the peripheral system (see Fig S3b in Guo et al. 2022). It is noted that 100% efficacy when administered via intravascular is crucial when it comes to therapy in humas, as the intracerebroventricular administration is not feasible in human newborns.

A second problem of this study is that the AAV is administered systemically (retroorbital route) to mice that are 10 days old (P10, see Fig. 3b in Gue et al. 2022), which is considered too late in this animal model, considering that GA-I disease turns severe in human babies when they reach an age of only 3 months. Therefore, the safety and efficacy of vectors carrying out the correct copy of GCDH needs to be proved at earlier times in mice, in order to ensure that their efficacy can be translated into clinical practice in infants of between 1-3 months of age, before the disease affects them.

The present invention solves the above mentioned problems by providing an a AAV vector that shows 100% efficacy when administered intravascularly in mice at day 1. Here we report the efficacy of AAV9-mediated systemic delivery of human GCDH under the control of the CAG promoter to prevent from the HLD-induced phenotype in *Gcdh* -/- mice. Neonatal delivery of AAV-GCDH restored GCDH expression and enzyme activity in liver and striatum that was maintained for at least 6 months. Reduction in glutaric acid, 3-OH glutaric acid and glutarylcarnitine accumulation were observed both one month and 6 months after therapy. AAV-GCDH significantly ameliorated the striatal neuropathology, minimizing vauole formation, gliosis and alterations in myelination. Prevention of brain injury was also observed in the MRI scans. Importantly, whereas 60% of *Gcdh* -/- mice succumbed from lysine overload, AAV-GCDH, administered at the same dose and route as the study of Guo et al. 2022 (1×10¹¹ vg, systemic route), prevented the aggressive phenotype and all mice survived to HLD (100% survival, see Fig. 8). Altogether, these results provide preclinical evidence to support AAV-GCDH gene therapy for GA-I.

Thus, in **a first aspect,** the present invention provides a nucleic acid or polynucleotide, also called herein the nucleic acid of the invention, comprising an expression cassette comprising:
(i) the nucleotide sequence of a chimeric promoter comprising the cytomegalovirus (CMV) enhancer, the chicken β-actin promoter, and a chimeric intron,
(ii) the nucleotide sequence encoding for the human glutaryl-CoA dehydrogenase enzyme (GCDH),
wherein (i) is operably linked to and regulates the expression of (ii).

Preferably, the nucleic acid or polynucleotide of the invention is comprised in the genome of an adeno-associated virus (AAV). Thus, in a preferred embodiment of the first aspect, an AAV genome is provided, also called herein the AAV genome of the invention, wherein said AAV genome comprises the nucleic acid or polynucleotide of the invention comprising an expression cassette comprising:
(i) the nucleotide sequence of a chimeric promoter comprising the cytomegalovirus (CMV) enhancer, the chicken β-actin promoter, and a chimeric intron,
(ii) the nucleotide sequence encoding for the human glutaryl-CoA dehydrogenase enzyme (GCDH),
wherein (i) is operably linked to and regulates the expression of (ii).

In an embodiment, the nucleic acid or the AAV of the invention are isolated and/or artificial. Each of the elements comprised in the nucleic acid or AAV genome of the invention are further defined below:
In an embodiment, the nucleotide sequence of the cytomegalovirus (CMV) enhancer comprises SEQ ID NO: 1 or a sequence which is at least 75%, 80%, 85%, 89%, 90%, 91%, 92%, 95%, 97%, 98%, 99%, or 100% identical over the full length of SEQ ID NO: 1. Preferably, the nucleotide sequence of the CMV enhancer comprises, consists, or consists essentially of SEQ ID NO: 1.

In an embodiment, the nucleotide sequence of the chicken β-actin promoter comprises SEQ ID NO: 2 or a sequence which is at least 75%, 80%, 85%, 89%, 90%, 91%, 92%, 95%, 97%, 98%, 99%, or 100% identical over the full length of SEQ ID NO: 2. Preferably, the nucleotide sequence of the chicken β-actin promoter comprises, consists, or consists essentially of SEQ ID NO: 2.

In an embodiment, the nucleotide sequence of the chimeric intron (chicken β-actin intron + rabbit β-globin intron) comprises SEQ ID NO: 3 or a sequence which is at least 75%, 80%, 85%, 89%, 90%, 91%, 92%, 95%, 97%, 98%, 99%, or 100% identical over the full length of SEQ ID NO: 3. Preferably, the nucleotide sequence of the chimeric intron comprises, consists, or consists essentially of SEQ ID NO: 3.

In an embodiment, the nucleotide sequence of the chimeric promoter (also called herein CAG promoter) comprises SEQ ID NO: 4 or a sequence which is at least 75%, 80%, 85%, 89%, 90%, 91%, 92%, 95%, 97%, 98%, 99%, or 100% identical over the full length of SEQ ID NO: 4. Preferably, the nucleotide sequence of the chicken β-actin promoter comprises, consists, or consists essentially of SEQ ID NO: 4.

In an embodiment, the nucleotide sequence encoding for the human GCDH comprises SEQ ID NO: 5 or 10 or a sequence which is at least 75%, 80%, 85%, 89%, 90%, 91%, 92%, 95%, 97%, 98%, 99%, or 100% identical over the full length of SEQ ID NO: 5 or 10. Preferably, the nucleotide sequence encoding for the human GCDH comprises, consists, or consists essentially of SEQ ID NO: 5 or 10. In an embodiment, the nucleotide sequence encoding for the human GCDH is operably linked to at least one regulatory sequence that drives the expression of the human GCDH.

In an embodiment, the amino acid sequence of the human GCDH encoded by the nucleotide sequence comprises SEQ ID NO: 11 or a sequence which is at least 75%, 80%, 85%, 89%, 90%, 91%, 92%, 95%, 97%, 98%, 99%, or 100% identical over the full length of SEQ ID NO: 11. Preferably, the amino acid sequence of the human GCDH comprises, consists, or consists essentially of SEQ ID NO: 11.

In an embodiment, the expression cassette further comprises a nucleotide sequence that encodes for a polyadenylation (poly(A)) signal in the 3' of the sequence encoding for the human GCDH, such that the expressed mRNA has a polyA tail. In some embodiments, the nucleotide sequence that encodes the poly(A) signal is a human Growth Hormone (hGH) poly(A) tail signal. In an embodiment, the nucleotide sequence of the hGH poly(A) tail signal comprises SEQ ID NO: 7 or a sequence which is at least 75%, 80%, 85%, 89%, 90%, 91%, 92%, 95%, 97%, 98%, 99%, or 100% identical over the full length of SEQ ID NO: 7. Preferably, the nucleotide sequence of the hGH poly(A) tail signal comprises, consists, or consists essentially of SEQ ID NO: 7.

In an embodiment, the expression cassette further comprises at least one posttranscriptional regulatory element operably linked to the nucleotide encoding for the human GCDH. In an embodiment, said posttranscriptional regulatory element is the WHV (woodchuck hepatitis virus) post-transcriptional regulatory element (WPRE). In an embodiment, the WPRE is downstream (3' of) the human GCDH coding sequence and upstream (5' of) the hGH poly(A) tail signal. In a preferred embodiment, the nucleotide sequence comprising the WPRE comprises SEQ ID NO: 6 or a sequence which is at least 75%, 80%, 85%, 89%, 90%, 91%, 92%, 95%, 97%, 98%, 99%, or 100% identical over the full length of SEQ ID NO: 6. Preferably, the nucleotide sequence of the WPRE comprises, consists, or consists essentially of SEQ ID NO: 6.

It is to be understood that all the embodiments described above for the different elements of the expression cassette (the chimeric promoter, the human GCDH gene, the WPRE element, and the poly(a) tail) can be combined with each other. Thus, in a preferred embodiment, the nucleic acid of the invention comprises an expression cassette comprising, preferably in the 5' to 3' direction,:
(i) the nucleotide sequence of a chimeric promoter comprising SEQ ID NO: 4, or a sequence with at least 95%, 96%, 97%, preferably 98%, 99.0%, 99.1%, 99.2%., 99.3%, 99.4%, 99.5%, 99.6%, 99.7%, 99.8%, 99.9% or 100% identity to SEQ ID NO: 4, and
(ii) the nucleotide sequence encoding for the human glutaryl-CoA dehydrogenase enzyme (GCDH) comprising the amino acid sequence of SEQ ID NO: 11, or a sequence with at least 95%, 96%, 97%, preferably 98%, 99.0%, 99.1%, 99.2%., 99.3%, 99.4%, 99.5%, 99.6%, 99.7%, 99.8%, 99.9% or 100% identity to SEQ ID NO: 11,
wherein (i) is operably linked to and regulates the expression of (ii), wherein the nucleic acid is preferably comprised in a AAV genome.

In a preferred embodiment, the nucleic acid of the invention comprises an expression cassette comprising, preferably in the 5' to 3' direction,:
(i) the nucleotide sequence of a chimeric promoter comprising SEQ ID NO: 4, or a sequence with at least 95%, 96%, 97%, preferably 98%, 99.0%, 99.1%, 99.2%., 99.3%, 99.4%, 99.5%, 99.6%, 99.7%, 99.8%, 99.9% or 100% identity to SEQ ID NO: 4,
(ii) the nucleotide sequence encoding for the human glutaryl-CoA dehydrogenase enzyme (GCDH) comprising the amino acid sequence of SEQ ID NO: 11, or a sequence with at least 95%, 96%, 97%, preferably 98%, 99.0%, 99.1%, 99.2%., 99.3%, 99.4%, 99.5%, 99.6%, 99.7%, 99.8%, 99.9% or 100% identity to SEQ ID NO: 11, and
(iii) the nucleotide sequence encoding for a hGH poly(A) tail,
wherein (i) is operably linked to and regulates the expression of (ii),and wherein the nucleic acid is preferably comprised in a AAV genome.

In a preferred embodiment, the nucleic acid of the invention comprises an expression cassette comprising, preferably in the 5' to 3' direction,:
(i) the nucleotide sequence of a chimeric promoter comprising SEQ ID NO: 4, or a sequence with at least 95%, 96%, 97%, preferably 98%, 99.0%, 99.1%, 99.2%., 99.3%, 99.4%, 99.5%, 99.6%, 99.7%, 99.8%, 99.9% or 100% identity to SEQ ID NO: 4,
(ii) the nucleotide sequence encoding for the human glutaryl-CoA dehydrogenase enzyme (GCDH) comprising the amino acid sequence of SEQ ID NO: 11, or a sequence with at least 95%, 96%, 97%, preferably 98%, 99.0%, 99.1%, 99.2%., 99.3%, 99.4%, 99.5%, 99.6%, 99.7%, 99.8%, 99.9% or 100% identity to SEQ ID NO: 11,
(iii) the nucleotide sequence encoding for the WHV (woodchuck hepatitis virus) posttranscriptional regulatory element (WPRE) and
(iv) the nucleotide sequence encoding for a hGH poly(A) tail,
wherein (i) and (iii) is operably linked to and regulates the expression of (ii), and wherein the nucleic acid is preferably comprised in a AAV genome.

In a preferred embodiment, the nucleic acid of the invention comprises an expression cassette comprising, preferably in the 5' to 3' direction,:
(i) the nucleotide sequence of a chimeric promoter comprising SEQ ID NO: 4,
(ii) the nucleotide sequence encoding for the human glutaryl-CoA dehydrogenase enzyme (GCDH) comprising the amino acid sequence of SEQ ID NO: 11,
(iii) the nucleotide sequence encoding for the WHV (woodchuck hepatitis virus) posttranscriptional regulatory element (WPRE) and
(iv) the nucleotide sequence encoding for a hGH poly(A) tail,
wherein (i) and (iii) is operably linked to and regulates the expression of (ii), and wherein the nucleic acid is preferably comprised in a AAV genome.

In a preferred embodiment, the nucleic acid of the invention comprises an expression cassette comprising, preferably in the 5' to 3' direction,:
(i) the nucleotide sequence of a chimeric promoter consisting of SEQ ID NO: 4,
(ii) the nucleotide sequence encoding for the human glutaryl-CoA dehydrogenase enzyme (GCDH) consisting of the amino acid sequence of SEQ ID NO: 11,
(iii) the nucleotide sequence encoding for the WHV (woodchuck hepatitis virus) posttranscriptional regulatory element (WPRE) and
(iv) the nucleotide sequence encoding for a hGH poly(A) tail,
wherein (i) and (iii) is operably linked to and regulates the expression of (ii), and wherein the nucleic acid is preferably comprised in a AAV genome.

In a preferred embodiment, the nucleic acid of the invention comprises an expression cassette comprising, preferably in the 5' to 3' direction,:
(i) the nucleotide sequence of a chimeric promoter comprising SEQ ID NO: 4, or a sequence with at least 95%, 96%, 97%, preferably 98%, 99.0%, 99.1%, 99.2%., 99.3%, 99.4%, 99.5%, 99.6%, 99.7%, 99.8%, 99.9% or 100% identity to SEQ ID NO: 4,
(ii) the nucleotide sequence encoding for the human glutaryl-CoA dehydrogenase enzyme (GCDH) comprising the amino acid sequence of SEQ ID NO: 11, or a sequence with at least 95%, 96%, 97%, preferably 98%, 99.0%, 99.1%, 99.2%., 99.3%, 99.4%, 99.5%, 99.6%, 99.7%, 99.8%, 99.9% or 100% identity to SEQ ID NO: 11,
(iii) the nucleotide sequence encoding for the WHV (woodchuck hepatitis virus) posttranscriptional regulatory element (WPRE) comprising SEQ ID NO: 6, or a sequence with at least 95%, 96%, 97%, preferably 98%, 99.0%, 99.1%, 99.2%., 99.3%, 99.4%, 99.5%, 99.6%, 99.7%, 99.8%, 99.9% or 100% identity to SEQ ID NO: 6, and
(iv) the nucleotide sequence encoding for a hGH poly(A) tail comprising SEQ ID NO: 7, or a sequence with at least 95%, 96%, 97%, preferably 98%, 99.0%, 99.1%, 99.2%., 99.3%, 99.4%, 99.5%, 99.6%, 99.7%, 99.8%, 99.9% or 100% identity to SEQ ID NO: 7,
wherein (i) and (iii) is operably linked to and regulates the expression of (ii), and wherein the nucleic acid is preferably comprised in a AAV genome.

In a preferred embodiment, the nucleic acid of the invention comprises an expression cassette comprising, preferably in the 5' to 3' direction,:
(i) the nucleotide sequence of a chimeric promoter comprising SEQ ID NO: 4, or a sequence with at least 95%, 96%, 97%, preferably 98%, 99.0%, 99.1%, 99.2%., 99.3%, 99.4%, 99.5%, 99.6%, 99.7%, 99.8%, 99.9% or 100% identity to SEQ ID NO: 4,
(ii) the nucleotide sequence encoding for the human glutaryl-CoA dehydrogenase enzyme (GCDH) comprising the sequence of SEQ ID NO: 5 or 10, or a sequence with at least 95%, 96%, 97%, preferably 98%, 99.0%, 99.1%, 99.2%., 99.3%, 99.4%, 99.5%, 99.6%, 99.7%, 99.8%, 99.9% or 100% identity to SEQ ID NO: 5 or 10,
(iii) the nucleotide sequence encoding for the WHV (woodchuck hepatitis virus) posttranscriptional regulatory element (WPRE) comprising SEQ ID NO: 6, or a sequence with at least 95%, 96%, 97%, preferably 98%, 99.0%, 99.1%, 99.2%., 99.3%, 99.4%, 99.5%, 99.6%, 99.7%, 99.8%, 99.9% or 100% identity to SEQ ID NO: 6, and
(iv) the nucleotide sequence encoding for a hGH poly(A) tail comprising SEQ ID NO: 7, or a sequence with at least 95%, 96%, 97%, preferably 98%, 99.0%, 99.1%, 99.2%., 99.3%, 99.4%, 99.5%, 99.6%, 99.7%, 99.8%, 99.9% or 100% identity to SEQ ID NO: 7,
wherein (i) and (iii) is operably linked to and regulates the expression of (ii), and wherein the nucleic acid is preferably comprised in a AAV genome.

In a preferred embodiment, the nucleic acid of the invention comprises an expression cassette comprising, preferably in the 5' to 3' direction,:
(i) the nucleotide sequence of a chimeric promoter comprising SEQ ID NO: 4,
(ii) the nucleotide sequence encoding for the human glutaryl-CoA dehydrogenase enzyme (GCDH) comprising the amino acid sequence of SEQ ID NO: 11,
(iii) the nucleotide sequence encoding for the WHV (woodchuck hepatitis virus) posttranscriptional regulatory element (WPRE) comprising SEQ ID NO: 6,
(iv) the nucleotide sequence encoding for a hGH poly(A) tail comprising SEQ ID NO: 7,
wherein (i) and (iii) is operably linked to and regulates the expression of (ii), and wherein the nucleic acid is preferably comprised in a AAV genome.

In a preferred embodiment, the nucleic acid of the invention comprises an expression cassette comprising, preferably in the 5' to 3' direction,:
(i) the nucleotide sequence of a chimeric promoter consisting of SEQ ID NO: 4,
(ii) the nucleotide sequence encoding for the human glutaryl-CoA dehydrogenase enzyme (GCDH) consisting of the amino acid sequence of SEQ ID NO: 11,
(iii) the nucleotide sequence encoding for the WHV (woodchuck hepatitis virus) posttranscriptional regulatory element (WPRE) consisting of SEQ ID NO: 6,
(iv) the nucleotide sequence encoding for a hGH poly(A) tail consisting of SEQ ID NO: 7,
wherein (i) and (iii) is operably linked to and regulates the expression of (ii), and wherein the nucleic acid is preferably comprised in a AAV genome.

In a preferred embodiment, the expression cassette comprises a nucleotide sequence having at least 75%, 80%, 85%, 86%, 87%, 88%, 89%, 90%, 91%, 92%, 93%, 94%, 95%, 96%, 97%, preferably 98%, 99.0%, 99.1%, 99.2%., 99.3%, 99.4%, 99.5%, 99.6%, 99.7%, 99.8%, 99.9% or 100% identity to SEQ ID NO: 9, or a reverse complementary sequence thereto, and is an expression cassette that is capable of expressing a functional human GCDH enzyme in human tissues. Preferably, the nucleotide sequence of the expression cassette comprises, consists, or consists essentially of SEQ ID NO: 9, or a reverse complementary sequence thereto. In an embodiment, the expression cassette comprising or consisting of SEQ ID NO: 9, or a sequence with at least 75%, 80%, 85%, 86%, 87%, 88%, 89%, 90%, 91%, 92%, 93%, 94%, 95%, 96%, 97%, preferably 98%, 99.0%, 99.1%, 99.2%., 99.3%, 99.4%, 99.5%, 99.6%, 99.7%, 99.8%, 99.9% or 100% identity to SEQ ID NO: 9 is comprised in an AAV genome.

Other elements may be included in the nucleic acid or in the AVV genome of the invention. In some embodiments, a recombinant self-complementary AAV (scAAV) genome is provided, comprising the expression cassette as defined above and one or more inverted terminal repeat (ITR). ITRs contains a number of cis-acting elements that are involved in the initiation of viral DNA replication. The ITRs may be placed at 5' and/or 3' end of the AAV genome. Inverted-terminal repeats (ITRs) from adeno-associated viruses (AAVs) constitute a vector when adjoined to the nucleic acid encoding a target protein because the ITRs will provide for the nucleic acid encoding the target protein to be packaged within an AAV virion. ITRs also provide other cis-acting functions for expression of the nucleic acid encoding the target protein in the host cell upon entry of the vector into the host cell. Such cis- acting functions of ITRs include aiding in concatemer formation for genomic insertion; initiation of second strand formation in the case of a single- stranded (ss) AAV (ssAAV) vector; or initiation of replication and transcription in the case of ssAAV and self-complementary (sc) AAV (scAAV) vectors. Preferably, the ITRs are from AAV2 genome.

In a preferred embodiment, the 5' ITR comprises a nucleotide sequence having at least 75%, 80%, 85%, 86%, 87%, 88%, 89%, 90%, 91%, 92%, 93%, 94%, 95%, 96%, 97%, 98%, 99.0%, 99.1%, 99.2%., 99.3%, 99.4%, 99.5%, 99.6%, 99.7%, 99.8%, 99.9% or 100% identity to SEQ ID NO: 8. Preferably, the nucleotide sequence of the 5' ITR comprises, consists, or consists essentially of SEQ ID NO: 8. In a preferred embodiment, the 3' ITR comprises a nucleotide sequence having at least 75%, 80%, 85%, 86%, 87%, 88%, 89%, 90%, 91%, 92%, 93%, 94%, 95%, 96%, 97%, 98%, 99.0%, 99.1%, 99.2%., 99.3%, 99.4%, 99.5%, 99.6%, 99.7%, 99.8%, 99.9% or 100% identity to SEQ ID NO: 12. Preferably, the nucleotide sequence of the 3' ITR comprises, consists, or consists essentially of SEQ ID NO: 12. In a preferred embodiment, the expression cassette defined above is placed between a 5' ITR and a 3' ITR, wherein preferably the 5' ITR and 3' ITR have a nucleotide sequence having at least 75%, 80%, 85%, 86%, 87%, 88%, 89%, 90%, 91%, 92%, 93%, 94%, 95%, 96%, 97%, 98%, 99.0%, 99.1%, 99.2%., 99.3%, 99.4%, 99.5%, 99.6%, 99.7%, 99.8%, 99.9% or 100% identity to SEQ ID NO: 8 and 12, respectively, and wherein the expression cassette preferably comprises a nucleotide sequence having at least 75%, 80%, 85%, 86%, 87%, 88%, 89%, 90%, 91%, 92%, 93%, 94%, 95%, 96%, 97%, 98%, 99.0%, 99.1%, 99.2%., 99.3%, 99.4%, 99.5%, 99.6%, 99.7%, 99.8%, 99.9% or 100% identity to SEQ ID NO: 9. Most preferably, the expression cassette defined above is placed between a 5' ITR and a 3' ITR, wherein the 5' ITR and 3' ITR have a nucleotide sequence having 100% identity to SEQ ID NO: 8 and 12, respectively, and wherein the expression cassette preferably comprises a nucleotide sequence having 100% identity to SEQ ID NO: 9.

Thus, in some embodiments, the nucleic acid or the AAV genome of the invention comprises, from 5' to 3', the following nucleotide sequences in the following order: at least one of the ITRs, the expression cassette as defined above, and at least one of the ITRs. Provided, thus, are expression cassettes that can be incorporated into an AAV virion for gene replacement expression of the target gene. In some embodiments, the rAAVs of the present invention are pseudotyped rAAVs. For example, a pseudotyped AAV vector containing the ITRs of serotype X encapsidated with the proteins of Y will be designated as AAVX/Y (e.g., AAV2/1 has the ITRs of AAV2 and the capsid of AAVI). In some embodiments, pseudotyped rAAVs may be useful for combining the tissue-specific targeting capabilities of a capsid protein from one AAV serotype with the viral DNA from another AAV serotype, thereby allowing targeted delivery of a transgene to a target tissue.

In an embodiment, the nucleic acid or the AAV genome of the invention is DNA, which may be double-stranded DNA or single-stranded DNA. In a preferred embodiment, the nucleic acid or the AAV genome is single stranded DNA, so that the complimentary copy of said genome needs to be generated within the infected cell.

In some embodiments, the nucleic acid or the AAV genome of the invention is packaged into a virion, also called herein the virion of the present invention. Hence, a second aspect of the present invention provides a recombinant virion comprising the nucleic acid or the AAV genome as defined in the first aspect or any of its embodiments. Preferably, the virion is a non-integrative virion. Preferably, the nucleic acid of the first aspect is comprised in a AAV genome, and the virion is a AVV virion. Preferably, the virion is a recombinant AAV virion comprising an AAV capsid protein and the nucleic acid or the AAV genome of the first aspect, that comprises the nucleotide sequence encoding for the human GCDH operably linked to regulatory elements as mentioned above. In an embodiment, the AAV capsid protein encapsulates the recombinant AAV genome.

In some embodiments, the AAV capsid protein comprises a VP1, a VP2, and a VP3. The capsid preferably has tropism for appropriate cells and tissues, such as, for example, nervous tissue, CNS, liver, etc. In some embodiments, the capsid protein comprises an AAV1 capsid protein, an AAV2 capsid protein, an AAV3 capsid protein, an AAV4 capsid protein, an AAV5 capsid protein, an AAV6 capsid protein, an AAV7 capsid protein, an AAV8 capsid protein, an AAV9 capsid protein, an AAV-DJ capsid protein, an AAV-DJ/8 capsid protein, an AAV-RhlO capsid protein, an AAV-retro capsid protein, an AAV-PHP.B capsid protein, an AAV8-PHP.eB capsid protein, or an AAV-PHP.S capsid protein. In a preferred embodiment, the AAV capsids is AAV9 capsid protein. Preferably, the AVV virion is made of capsid proteins of the serotype 9 adeno-associated virus (AAV9), and the nucleic acid sequence contained in the capsid is flanked at both ends by internal terminal repeats corresponding to serotype 2 adeno-associated viruses.

In some embodiments, the nucleic acid or virions described herein can be modified and/or selected to enhance the targeting of the virions to a target tissue (e.g., CNS). Nonlimiting methods of modifications and/or selections include AAV capsid serotypes (e.g., AAV9), tissue-specific promoters, and/or targeting peptides. In some embodiments, the nucleic acids and AAVs disclosed herein can comprise AAV capsid serotypes with enhanced targeting to CNS tissues (e.g., AAV9). In some embodiments, the nucleic acids and AAVs described herein can comprise AAV capsid serotypes with enhanced targeting to CNS tissues and tissue-specific promoters. While AAV9 targets CNS tissue, the AAV9 vectors may also transduce other non-CNS tissues and, thus, the transgenes, under the control of a promoter such as the CAG promoter may be expressed both in the CNS and other tissues outside the CNS.

In an embodiment, a plasmid comprising the nucleic acid or the AAV genome of the invention is used to prepare and generate the recombinant virion, preferably AAV virion, of the second aspect. The plasmids provided for said purpose generally have an origin of replication and selectable markers to permit reproduction of the plasmid and use in host cells for generating the recombinant viral particles described herein. In some embodiments, the plasmid further comprises a bacterial expressing region. In some embodiments, the bacterial expressing region comprises a bacterial promoter and a nucleic acid that encodes a bacterial selecting region. In some embodiments, the nucleic acid that encodes the bacterial selecting region is operably linked to the bacterial promoter. In some embodiments, the nucleic acid that encodes the bacterial selecting region comprises a nucleic acid that encodes an antibiotic resistance gene or protein. In some embodiments, the plasmid further comprises an origin of replication. In some embodiments, the origin of replication comprises a CMV origin of replication (ori).

Methods for obtaining recombinant virions having a desired capsid protein are known in the art. Typically, for the case of AAV virions, the methods involve culturing a host cell which contains a nucleic acid sequence encoding an AAV capsid protein or fragment thereof; a functional rep gene; sufficient helper functions to permit packaging of the recombinant AAV genome into the AAV capsid proteins; and a plasmid comprising the AAV genome. Typically, capsid proteins are structural proteins encoded by the cap gene of an AAV. In some embodiments, the capsid protein comprises VP1, VP2, and VP3, said VP1, VP2, and VP3 and are transcribed from a single cap gene via alternative splicing. In some embodiments, upon translation, capsid proteins form a spherical 60-mer protein shell around the AAV genome. Capsid proteins protect the AAV genome, deliver said genome and/or interact with a host cell.

In some embodiments, components to be cultured in the host cell to package a recombinant AAV genome in an AAV capsid can be provided to the host cell in trans. Alternatively, any one or more of the required components (e.g., recombinant AAV genome, rep sequences, cap sequences, and/or helper functions) can be provided by a stable host cell which has been engineered to contain one or more of the required components. In particular embodiments, provided are host cells comprising the recombinant AAV constructs or plasmids comprising the expression cassette of interest, a plasmid providing the AAV rep and cap gene sequences, and a construct providing adenoviral helper proteins as needed to produce the recombinant viral particle.

The recombinant AAV genome, rep sequences, cap sequences, and helper functions useful for producing the rAAV virion described herein can be delivered to the packaging host cell using any appropriate genetic element (vector, e.g. plasmid). The selected genetic element can be delivered by any suitable method. The methods used to construct any of compositions disclosed herein are known to those with skill in nucleic acid manipulation and include genetic engineering, recombinant engineering, and synthetic techniques. Similarly, methods of generating rAAV virions are well known and the selection of a suitable method is not a limitation on the present disclosure. Typically, the recombinant AAV virions can be produced by transfecting a host cell with the recombinant AAV genome to be packaged into AAV particles, an AAV helper function vector, and an accessory function vector. An AAV helper function vector encodes the "AAV helper function" sequences (i.e., rep and cap), which function in trans for productive AAV replication and encapsidation with the cap gene encoding the capsid proteins of desired serotype, for example, encoding the AAV9 capsid. In some embodiments, the AAV helper function vector can support efficient AAV virion production without generating any detectable wild-type AAV virions (i.e., AAV virions containing functional rep and cap genes). The accessory function vector encodes nucleotide sequences for non-AAV derived viral and/or cellular functions upon which AAV is dependent for replication (i.e., "accessory functions"). The accessory functions include those functions required for AAV replication, including, without limitation, those moieties involved in activation of AAV gene transcription, stage specific AAV mRNA splicing, AAV DNA replication, synthesis of cap expression products, and AAV capsid assembly.

In some embodiments, the virion of the second aspect or any of its embodiments, which is preferably a AVV virion, is capable to transduce eukaryotic cells, preferably human cells. Thus, in a **third** aspect, the present invention relates to a host cell, preferably a mammalian cell or a human cell, or a substantially pure population of cells comprising (e.g., transfected or transduced) the nucleic acid, the AAV genome, or the virion as defined in the first or second aspects, or any of their embodiments. Preferably, the cells or substantially pure population of cells comprise the AAV virion or the AAV genome of the invention. In an embodiment of the third aspect, the host cell or the substantially pure population of cells are brain cells, such as neurons or glial cells.

In some embodiments, the cell or the substantially pure population of cells according to the third aspect or any of its embodiments, are transduced with the virion according to the second aspect or any of its embodiments, either *in vivo, in vitro,* or ex *vivo.* Said cells may be transduced with the AAV virions, thereby introducing the AAV genome inside said cells. The cell or the substantially pure population of cells according to the third aspect can thus be used as a recipient of the AAV genome and/or the AAV virion of the present invention.

In a **fourth aspect,** the present invention provides a pharmaceutical composition comprising the nucleic acid or the virion and/or the cell as defined in the first, second, or third aspects, respectively, or in any of their embodiments, and a pharmaceutically acceptable carrier or diluent. Preferably, the pharmaceutical composition comprises the AAV genome as defined in the first aspect, the AAV virion as defined in the second aspect, and/or the cell or substantially pure population of cells as defined in the third aspect, or in any of their embodiments. As used herein, "pharmaceutically acceptable carrier" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed are also known in the art.

The pharmaceutical composition as described herein may also contain other substances, including, but not limiting to, cryoprotectants, lyoprotectants, surfactants, bulking agents, antioxidants, and stabilizing agents. In some embodiments, the pharmaceutical composition may be lyophilized.

The pharmaceutical composition may be prepared for or be suitable for oral, sublingual, buccal, intravenous, intravascular, intramuscular, subcutaneous, intraperitoneal, conjunctival, rectal, transdermal, intrathecal, topical and/or inhalation-mediated administration. In a preferred embodiment, the pharmaceutical composition may be prepared for or be suitable for intravenous and/or intravascular administration. In another embodiment, the pharmaceutical composition may be a solution which is suitable for sublingual, buccal and/or inhalation-mediated administration routes. In an alternative embodiment, the pharmaceutical composition may be a gel or solution which is suitable for intrathecal, intraestriatal or intracerebroventricular administration. In an alternative embodiment, the pharmaceutical composition may be an aerosol which is suitable for inhalation-mediated administration. In another embodiment, the pharmaceutical composition may be prepared for administration in the cisterna magna or in the cerebrospinal fluid. Additionally, in other embodiments compatible with the teachings herein, the pharmaceutical composition can be prepared to be delivered directly to the site of the adverse cellular population, thereby increasing the exposure of the diseased tissue to the therapeutic agent.

The agents described herein may, in some embodiments, be assembled into pharmaceutical or diagnostic or research kits to facilitate their use in therapeutic, diagnostic or research applications. A kit may include one or more containers housing the components of the invention and instructions for use. Specifically, such kits may include one or more agents described herein, along with instructions describing the intended application and the proper use of these agents. In certain embodiments agents in a kit may be in a pharmaceutical formulation and dosage suitable for a particular application and for a method of administration of the agents. In some embodiments, the pharmaceutical composition is provided in a kit. The kit can further comprise a container housing a pharmaceutically acceptable carrier. For example, a kit can comprise one container housing a the nucleic acid of the first aspect or the virion of the second aspect and a second container housing a buffer suitable for injection of the nucleic acid or the virion into a subject. In an embodiment, the container can be a syringe. The kits disclosed herein can also contain any one or more of the components described herein in one or more containers. In some embodiments, the kits can include instructions for mixing one or more components of the kit and/or isolating and mixing a sample and applying to a subject. The kits can include a container housing agents described herein. The agents can be in the form of a liquid, gel or solid (powder).

The agents can be prepared sterilely, packaged in syringe and shipped refrigerated. Alternatively, it can be housed in a vial or other container for storage. A second container can have other agents prepared sterilely. Alternatively, the kits can include the active agents premixed and shipped in a syringe, vial, tube, or other container. The kits can have one or more or all of the components required to administrate the therapeutic agents to an animal, such as a syringe, topical application devices, or a needle tubing and bag.

In a **fifth aspect,** the present invention provides the nucleic acid or the AAV genome according to the first aspect or any of its embodiment, the virion according to the second aspect or any of its embodiments, the cell or the substantially pure population of cells according to the third aspect or any of its embodiments, or the pharmaceutical composition of the fourth aspect or any of its embodiments for use as a medicament or for use in therapy. Preferably, a fifth aspect provides the AAV genome or the AAV virion as defined in the first or second aspects, respectively, for use in medicine or as a medicament.

In a **sixth aspect,** the present invention provides the nucleic acid, preferably AAV genome, according to the first aspect or any of its embodiment, the virion, preferably the AAV virion, according to the second aspect or any of its embodiments, the cell or the substantially pure population of cells according to the third aspect or any of its embodiments, or the pharmaceutical composition of the fourth aspect or any of its embodiments, for use in the treatment of Glutaric aciduria type I (GA-I), said use preferably comprising administering to the subject said nucleic acid or AAV genome, said virion, said host cell or population of cells, or said composition.

The present invention also provides methods of treating, preventing, or ameliorating the GA-I disorder, or GA-I disease progression, in a subject in need thereof comprising administering to the subject a therapeutically effective amount of the nucleic acid (preferably the AAV genome), the virion (preferably AAV virion), the cell or substantially pure population of cells, or the pharmaceutical composition according to the first, second, third or fourth aspects, respectively, or any of their embodiments. In some embodiments, a method of reducing a disease condition in a subject, who suffers from GA-I, is provided. In some embodiments, the method comprises contacting the nucleic acid or virion, the cell or population of cells, or the pharmaceutical composition as defined in the above aspects, or an effective amount thereof, with at least one of the liver, the spleen, a muscle, a kidney, the blood, a lens, an eye, the cerebellum, the brainstem, the basal ganglia, the hypothalamus, the preoptic area, a hippocampus, a striatum, a cortex, a motor cortex, a prefrontal cortex, a somatosensory cortex, a temporal cortex, a visual cortex, an occipital lobe, a temporal lobe, a parietal lobe, a frontal lobe, a bone, a reproductive organ, an ovary, a testis, a skin surface, a prostate, a uterus, or a pancreas of the subject.

In some embodiments, provided is a method to restore GCDH enzyme expression, the method comprising administering to a subject in need thereof a therapeutically effective amount of an agent according to any of the previous aspects, preferably the AAV virion or the pharmaceutical composition of the invention, comprising a nucleic acid sequence encoding the human GCDH, wherein expression of the nucleic acid sequence generates a functional GCDH.

In an embodiment, the treatment of GA-I includes: (i) preventing the undesired physiological change and/or pathological condition from occurring in a subject that can be predisposed to a disease or disorder characterized by lysine catabolism dysfunction and/or malfunction (such as GA-I) but has not yet been diagnosed as having it; (ii) inhibiting the physiological change and/or pathological condition (a disease or disorder characterized by lysine catabolism dysfunction and/or malfunction (such as GA-I)); or (iii) relieving the physiological change and/or pathological condition, i.e., causing regression of a disease or disorder characterized by lysine catabolism dysfunction and/or malfunction (such as GA-I).

In an embodiment, the administration of the nucleic acid, preferably AAV genome, according to the first aspect or any of its embodiment, the virion, preferably the AAV virion, according to the second aspect or any of its embodiments, the cell or the substantially pure population of cells according to the third aspect or any of its embodiments, or the pharmaceutical composition of the fourth aspect or any of its embodiments is for preventing GA-I. "Prevent" or "preventing" or "prevention" refers to precluding, averting, obviating, forestalling, stopping, or hindering something from happening, especially by advance action. In an embodiment, preventing lysine catabolism dysfunction and/or malfunction (such as GA-I) or the worsening of lysine catabolism dysfunction and/or malfunction (such as GA-1) is intended. The words "prevent" and "preventing" and "prevention" also refer to prophylactic or preventative measures for protecting or precluding a subject (e.g., an individual) not having lysine catabolism dysfunction and/or malfunction (such as GA-I) related complication from progressing to that complication. Preferably, the treatment can improve and/or diminish and/or ameliorate one or more symptoms associated GA-I in a subject. In an embodiment, the nucleic acid sequence of the invention encoding a GA-I can improve and/or diminish and/or ameliorate one or more pathologies associated with GA-I in a subject.

In some embodiments, successful treatment is determined when one or more of the following is detected: alleviation or amelioration of one or more of symptoms of the treated subject's disease, disorder, or condition, diminishment of extent of the subject's disease, disorder, or condition, stabilized (i.e., not worsening) state of a disease, disorder, or condition, delay or slowing of the progression of the disease, disorder, or condition, and amelioration or palliation of the disease, disorder, or condition. In some embodiments, success of treatment is determined by detecting the presence repaired target polynucleotide in one or more cells, tissues, or organs isolated from the subject. In some embodiments, success of treatment is determined by detecting the presence of human GCDH enzyme encoded by the polynucleotide of the first aspect in one or more cells, tissues, or organs isolated from the subject.

In an embodiment, the uses or treatment described herein promotes or is capable of enhancing the expression of human GCDH compared to a control. In some embodiments, administering the pharmaceutical compositions, the cells or the nucleic acids or virion described herein to a subject promotes expression of GCDH by at least 1.1, 1.2, 1.3, 1.4, 1.5, 1.6, 1.7, 1.8, 1.9, or 2-fold compared to a control. In an embodiment, administering the nucleic acids and/or virions and/or cells or pharmaceutical composition described herein to a subject promotes expression of GCDH in a subject (e.g., promotes expression of GCDH in the CNS of a subject) by between a 10%, 20%, 30%, 40%, 50%, 60%, 70%, 80%, 90%, or 100% increase compared to a control. Preferably, the control is an untreated cell or population of cells presenting GA-I phenotype.

Preferably, the administration of a nucleic acid or AAV genome, virion, the host cell or the substantially pure population of cells, or the composition provided herein does not induce an immune response in a subject in need thereof or induces a very low immune response in said subject. The administration can be local or, preferably, systemic. The administration may be parenteral. Preferably, the administration is sublingual, buccal, intravenous, intraarterial, intravascular, intramuscular, intrathecal, subcutaneous, intraperitoneal, conjunctival, rectal, transdermal, intrathecal, topical and/or inhalation-mediated administration. In another embodiment, administration is sublingual, buccal and/or inhalation-mediated administration routes. The intravenous, intravascular o intraarterial form of parenteral administration is preferred. In an embodiment, a form for administration would be a solution for injection, in particular for intravenous or intraarterial injection or drip. In other embodiments compatible with the teachings herein, the administration is directly to the site of the adverse cellular population, thereby increasing the exposure of the diseased tissue to the therapeutic agent.

In an embodiment, the nucleic acid or AAV genome, virion, the cell or substantially pure population of cells, or the pharmaceutical composition according to the first, second, third or fourth aspects, respectively, or any of their embodiments are administered as a single dose or multiple doses. In some embodiments, the doses are administered once or divided into multiple sub-dose, e.g., two sub-doses, three sub-doses, four sub-doses, five sub-doses, six sub-doses, or more than six sub-doses. In some embodiments, more than one genome, virion or cell is administered. Most preferably, the administration is as a single dose.

In an embodiment, the nucleic acid or AAV genome, the virion, the cell or substantially pure population of cells, or the pharmaceutical composition according to the first, second, third or fourth aspects, respectively, or any of their embodiments are administrated repeated at least twice, at least three times, at least four times, at least five times, at least six times, at least seven times, at least eight times, at least nine times, or at least ten times. Multiple doses can be administered continuously or at specific time intervals. Intervals between single dosages can be daily, weekly, monthly or yearly. Intervals can also be irregular depending on the development of the disease or the curative effects of said vectors.

As used herein, the phrase "subject in need thereof" includes subjects, such as mammalian subjects, that would benefit from the administration. In particular, the mammal may be a mouse, rat, rabbit, pig, horse, sheep, cow, domestic cat or dog, or a human. Usually, the subject is a human but non-human mammals including transgenic mammals can also be treated. In one embodiment, the subjects include, but are not limited to, individuals with GA-I. Preferably, the subject is a pediatric subject. Preferably, the nucleic acid or the AAV genome, the virion, the cell, or the pharmaceutical composition described herein is administrated to a paediatric subject, wherein the paediatric subject is a human neonate (birth to age less than 1 month) or an human infant (age 1 month to less than 2 years). Most preferably, the paediatric subject is a human of between 1-3 month of age, preferably younger than 3 months old.

In an embodiment, the subject has or is suspected of having a disease or disorder associated with low levels of GCDH expression (e.g., GCDH deficiency), preferably GA-1. In some embodiments, the subject is deficient in GCDH, as encoded by and expressed from the genome of the subject, due for example to an autosomal recessive inheritance of two defective GA GCDH genes.

The uses of the nucleic acid or AAV genome, the virion, the cell or substantially pure population of cells, or the pharmaceutical composition according to the first, second, third or fourth aspects, respectively, or any of their embodiments described herein are preferably in a subject of need thereof.

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited

### SEQUENCE LISTING

SEQ ID NO: 1: nucleotide sequence of the cytomegalovirus (CMV) enhancer: 
CGTTACATAACTTACGGTAAATGGCCCGCCTGGCTGACCGCCCAACGACCCCCGCCCATT
GACGTCAATAATGACGTATGTTCCCATAGTAACGCCAATAGGGACTTTCCATTGACGTCAAT
GGGTGGACTATTTACGGTAAACTGCCCACTTGGCAGTACATCAAGTGTATCATATGCCAAG
TACGCCCCCTATTGACGTCAATGACGGTAAATGGCCCGCCTGGCATTATGCCCAGTACATG
ACCTTATGGGACTTTCCTACTTGGCAGTACATCTACGTATTAGTCATCGCTATTACCATG
**SEQ** ID NO: 2: nucleotide sequence of the chicken β-actin promoter: 
TCGAGGTGAGCCCCACGTTCTGCTTCACTCTCCCCATCTCCCCCCCCTCCCCACCCCCAA
TTTTGTATTTATTTATTTTTTAATTATTTTGTGCAGCGATGGGGGCGGGGGGGGGGGGGGC
GCGCGCCAGGCGGGGCGGGGCGGGGCGAGGGGCGGGGCGGGGCGAGGCGGAGAGGT
GCGGCGGCAGCCAATCAGAGCGGCGCGCTCCGAAAGTTTCCTTTTATGGCGAGGCGGCG
GCGGCGGCGGCCCTATAAAAAGCGAAGCGCGCGGCGGGCG
**SEQ** ID NO: 3: nucleotide sequence of the chimeric intron (chicken β-actin intron + rabbit β-globin intron) 
GGAGTCGCTGCGTTGCCTTCGCCCCGTGCCCCGCTCCGCGCCGCCTCGCGCCGCCCGC
CCCGGCTCTGACTGACCGCGTTACTCCCACAGGTGAGCGGGCGGGACGGCCCTTCTCCT
CCGGGCTGTAATTAGCGCTTGGTTTAATGACGGCTCGTTTCTTTTCTGTGGCTGCGTGAAA
GCCTTAAAGGGCTCCGGGAGGGCCCTTTGTGCGGGGGGGAGCGGCTCGGGGGGTGCGT
GCGTGTGTGTGTGCGTGGGGAGCGCCGCGTGCGGCCCGCGCTGCCCGGCGGCTGTGAG
CGCTGCGGGCGCGGCGCGGGGCTTTGTGCGCTCCGCGTGTGCGCGAGGGGAGCGCGG
CCGGGGGCGGTGCCCCGCGGTGCGGGGGGGCTGCGAGGGGAACAAAGGCTGCGTGCG
GGGTGTGTGCGTGGGGGGGTGAGCAGGGGGTGTGGGCGCGGCGGTCGGGCTGTAACCC
CCCCCTGCACCCCCCTCCCCGAGTTGCTGAGCACGGCCCGGCTTCGGGTGCGGGGCTCC
GTACGGGGCGTGGCGCGGGGCTCGCCGTGCCGGGCGGGGGGTGGCGGCAGGTGGGGG
TGCCGGGCGGGGCGGGGCCGCCTCGGGCCGGGGAGGGCTCGGGGGAGGGGCGCGGC
GGCCCCGGAGCGCCGGCGGCTGTCGAGGCGCGGCGAGCCGCAGCCATTGCCTTTTATG
GTAATCGTGCGAGAGGGCGCAGGGACTTCCTTTGTCCCAAATCTGTGCGGAGCCGAAATC
TGGGAGGCGCCGCCGCACCCCCTCTAGCGGGCGCGGGGCGAAGCGGTGCGGCGCCGG
CAGGAAGGAAATGGGCGGGGAGGGCCTTCGTGCGTCGCCGCGCCGCCGTCCCCTTCTCC
CTCTCCAGCCTCGGGGCTGTCCGCGGGGGGACGGCTGCCTTCGGGGGGGACGGGGCAG
GGCGGGGTTCGGCTTCTGGCGTGTGACCGGCGGCTCTAGAGCCTCTGCTAACCATGTTCA
TGCCTTCTTCTTTTTCCTACAG
**SEQ** ID NO: 4: chimeric promoter (also called herein CAG promoter): nucleotide sequence of the cytomegalovirus (CMV) enhancer + nucleotide sequence of the chicken β-actin promoter + chimeric intron . 
CGTTACATAACTTACGGTAAATGGCCCGCCTGGCTGACCGCCCAACGACCCCCGCCCATT
GACGTCAATAATGACGTATGTTCCCATAGTAACGCCAATAGGGACTTTCCATTGACGTCAAT
GGGTGGACTATTTACGGTAAACTGCCCACTTGGCAGTACATCAAGTGTATCATATGCCAAG
TACGCCCCCTATTGACGTCAATGACGGTAAATGGCCCGCCTGGCATTATGCCCAGTACATG
ACCTTATGGGACTTTCCTACTTGGCAGTACATCTACGTATTAGTCATCGCTATTACCATGTC
GAGGTGAGCCCCACGTTCTGCTTCACTCTCCCCATCTCCCCCCCCTCCCCACCCCCAATTT
TGTATTTATTTATTTTTTAATTATTTTGTGCAGCGATGGGGGCGGGGGGGGGGGGGGCGC
GCGCCAGGCGGGGCGGGGCGGGGCGAGGGGCGGGGCGGGGCGAGGCGGAGAGGTGC
 
GGCGGCAGCCAATCAGAGCGGCGCGCTCCGAAAGTTTCCTTTTATGGCGAGGCGGCGGC
GGCGGCGGCCCTATAAAAAGCGAAGCGCGCGGCGGGCGGGAGTCGCTGCGTTGCCTTC
GCCCCGTGCCCCGCTCCGCGCCGCCTCGCGCCGCCCGCCCCGGCTCTGACTGACCGCG
TTACTCCCACAGGTGAGCGGGCGGGACGGCCCTTCTCCTCCGGGCTGTAATTAGCGCTTG
GTTTAATGACGGCTCGTTTCTTTTCTGTGGCTGCGTGAAAGCCTTAAAGGGCTCCGGGAGG
GCCCTTTGTGCGGGGGGGAGCGGCTCGGGGGGTGCGTGCGTGTGTGTGTGCGTGGGGA
GCGCCGCGTGCGGCCCGCGCTGCCCGGCGGCTGTGAGCGCTGCGGGCGCGGCGCGGG
GCTTTGTGCGCTCCGCGTGTGCGCGAGGGGAGCGCGGCCGGGGGCGGTGCCCCGCGGT
GCGGGGGGGCTGCGAGGGGAACAAAGGCTGCGTGCGGGGTGTGTGCGTGGGGGGGTG
AGCAGGGGGTGTGGGCGCGGCGGTCGGGCTGTAACCCCCCCCTGCACCCCCCTCCCCG
AGTTGCTGAGCACGGCCCGGCTTCGGGTGCGGGGCTCCGTACGGGGCGTGGCGCGGGG
CTCGCCGTGCCGGGCGGGGGGTGGCGGCAGGTGGGGGTGCCGGGCGGGGCGGGGCC
GCCTCGGGCCGGGGAGGGCTCGGGGGAGGGGCGCGGCGGCCCCGGAGCGCCGGCGG
CTGTCGAGGCGCGGCGAGCCGCAGCCATTGCCTTTTATGGTAATCGTGCGAGAGGGCGC
AGGGACTTCCTTTGTCCCAAATCTGTGCGGAGCCGAAATCTGGGAGGCGCCGCCGCACCC
CCTCTAGCGGGCGCGGGGCGAAGCGGTGCGGCGCCGGCAGGAAGGAAATGGGCGGGG
AGGGCCTTCGTGCGTCGCCGCGCCGCCGTCCCCTTCTCCCTCTCCAGCCTCGGGGCTGT
CCGCGGGGGGACGGCTGCCTTCGGGGGGGACGGGGCAGGGCGGGGTTCGGCTTCTGG
CGTGTGACCGGCGGCTCTAGAGCCTCTGCTAACCATGTTCATGCCTTCTTCTTTTTCCTAC
AG
**SEQ** ID NO: 5: GCDH cDNA 
ATGGCCCTGAGAGGCGTCTCCGTGCGGCTGCTGAGCCGCGGACCCGGCCTGCACGTCCT
TCGCACGTGGGTCTCGTCGGCGGCGCAGACCGAGAAAGGCGGGAGAACACAGAGCCAAC
TGGCTAAGTCCTCGCGTCCCGAGTTTGACTGGCAGGACCCGCTGGTGCTGGAGGAGCAG
CTGACCACAGATGAGATCCTCATCAGGGACACCTTCCGCACCTACTGCCAGGAGAGACTC
ATGCCTCGCATCCTGTTGGCCAATCGCAACGAAGTTTTTCATCGGGAGATCATTTCGGAGA
TGGGGGAGTTGGGTGTGCTGGGCCCCACCATCAAAGGATATGGCTGTGCTGGGGTTTCGT
CTGTGGCCTATGGGCTCCTGGCCCGAGAGCTGGAGCGGGTGGACAGTGGCTACAGGTCG
GCGATGAGTGTCCAGTCCTCCCTCGTCATGCACCCTATCTATGCCTATGGCAGCGAGGAA
CAGCGGCAGAAGTACCTGCCCCAGCTGGCCAAGGGGGAGCTCCTGGGCTGCTTCGGGCT
CACAGAGCCCAACAGCGGAAGTGACCCCAGCAGCATGGAGACCAGAGCCCACTACAACT
CATCCAACAAGAGCTACACCCTCAATGGGACCAAGACCTGGATCACGAACTCGCCTATGG
CCGATCTGTTTGTAGTGTGGGCTCGGTGTGAAGATGGCTGCATTCGGGGCTTCCTGCTGG
AGAAGGGGATGCGGGGTCTCTCGGCCCCCAGGATCCAGGGCAAGTTCTCGCTGCGGGCC
TCAGCCACAGGCATGATCATCATGGACGGTGTGGAGGTGCCAGAGGAGAATGTGCTCCCT
GGTGCATCCAGCCTGGGGGGTCCCTTCGGCTGCCTGAACAACGCCCGGTACGGCATCGC
GTGGGGCGTGCTTGGAGCTTCGGAGTTCTGCTTGCACACAGCCCGGCAGTACGCCCTCG
ACAGGATGCAGTTTGGTGTCCCACTGGCCAGGAACCAGCTGATTCAGAAGAAGCTGGCAG
ACATGCTCACTGAGATTACCCTGGGCCTTCACGCCTGCCTGCAGCTCGGCCGCTTGAAGG
ACCAGGACAAGGCTGCCCCCGAGATGGTTTCTCTGCTGAAGAGGAATAACTGTGGGAAAG
CCCTGGACATCGCCCGCCAGGCCCGAGACATGCTGGGGGGGAATGGGATTTCTGACGAG
TATCACGTGATCCGGCACGCCATGAACCTGGAGGCCGTGAACACCTACGAAGGTACACAT
GACATTCACGCCCTGATCCTTGGGAGAGCTATCACGGGAATCCAGGCGTTCACGGCCAGC
AAGTTGTGA
**SEQ** ID NO: 6: WHV (woodchuck hepatitis virus) post-transcriptional regulatory element (WPRE): 
AATCAACCTCTGGATTACAAAATTTGTGAAAGATTGACTGGTATTCTTAACTATGTTGCTCCT
TTTACGCTATGTGGATACGCTGCTTTAATGCCTTTGTATCATGCTATTGCTTCCCGTATGGC
TTTCATTTTCTCCTCCTTGTATAAATCCTGGTTGCTGTCTCTTTATGAGGAGTTGTGGCCCG
TTGTCAGGCAACGTGGCGTGGTGTGCACTGTGTTTGCTGACGCAACCCCCACTGGTTGGG
GCATTGCCACCACCTGTCAGCTCCTTTCCGGGACTTTCGCTTTCCCCCTCCCTATTGCCAC
 
GGCGGAACTCATCGCCGCCTGCCTTGCCCGCTGCTGGACAGGGGCTCGGCTGTTGGGCA
CTGACAATTCCGTGGTGTTGTCGGGGAAATCATCGTCCTTTCCTTGGCTGCTCGCCTATGT
TGCCACCTGGATTCTGCGCGGGACGTCCTTCTGCTACGTCCCTTCGGCCCTCAATCCAGC
GGACCTTCCTTCCCGCGGCCTGCTGCCGGCTCTGCGGCCTCTTCCGCGTCTTCGCCTTCG
CCCTCAGACGAGTCGGATCTCCCTTTGGGCCGCCTCCCCGC
**SEQ ID NO: 7:** hGH polyadenylation (poly(A)) signal: 
GGGTGGCATCCCTGTGACCCCTCCCCAGTGCCTCTCCTGGCCCTGGAAGTTGCCACTCCA
GTGCCCACCAGCCTTGTCCTAATAAAATTAAGTTGCATCATTTTGTCTGACTAGGTGTCCTT
CTATAATATTATGGGGTGGAGGGGGGTGGTATGGAGCAAGGGGCAAGTTGGGAAGACAAC
CTGTAGGGCCTGCGGGGTCTATTGGGAACCAAGCTGGAGTGCAGTGGCACAATCTTGGCT
CACTGCAATCTCCGCCTCCTGGGTTCAAGCGATTCTCCTGCCTCAGCCTCCCGAGTTGTTG
GGATTCCAGGCATGCATGACCAGGCTCAGCTAATTTTTGTTTTTTTGGTAGAGACGGGGTT
TCACCATATTGGCCAGGCTGGTCTCCAACTCCTAATCTCAGGTGATCTACCCACCTTGGCC
TCCCAAATTGCTGGGATTACAGGCGTGAACCACTGCTCCCTTCCCTGTCCTT
**SEQ ID NO: 8:** ITR 5' sequences 
CCTGCAGGCAGCTGCGCGCTCGCTCGCTCACTGAGGCCGCCCGGGCAAAGCCCGGGCG
TCGGGCGACCTTTGGTCGCCCGGCCTCAGTGAGCGAGCGAGCGCGCAGAGAGGGAGTG
GCCAACTCCATCACTAGGGGTTCCT
**SEQ ID NO: 9:** expression cassette (CAG chimeric promoter+GCDH+WPRE+PolyA): 
CGTTACATAACTTACGGTAAATGGCCCGCCTGGCTGACCGCCCAACGACCCCCGCCCATT
GACGTCAATAATGACGTATGTTCCCATAGTAACGCCAATAGGGACTTTCCATTGACGTCAAT
GGGTGGACTATTTACGGTAAACTGCCCACTTGGCAGTACATCAAGTGTATCATATGCCAAG
TACGCCCCCTATTGACGTCAATGACGGTAAATGGCCCGCCTGGCATTATGCCCAGTACATG
ACCTTATGGGACTTTCCTACTTGGCAGTACATCTACGTATTAGTCATCGCTATTACCATGTC
GAGGTGAGCCCCACGTTCTGCTTCACTCTCCCCATCTCCCCCCCCTCCCCACCCCCAATTT
TGTATTTATTTATTTTTTAATTATTTTGTGCAGCGATGGGGGCGGGGGGGGGGGGGGCGC
GCGCCAGGCGGGGCGGGGCGGGGCGAGGGGCGGGGCGGGGCGAGGCGGAGAGGTGC
GGCGGCAGCCAATCAGAGCGGCGCGCTCCGAAAGTTTCCTTTTATGGCGAGGCGGCGGC
GGCGGCGGCCCTATAAAAAGCGAAGCGCGCGGCGGGCGGGAGTCGCTGCGTTGCCTTC
GCCCCGTGCCCCGCTCCGCGCCGCCTCGCGCCGCCCGCCCCGGCTCTGACTGACCGCG
TTACTCCCACAGGTGAGCGGGCGGGACGGCCCTTCTCCTCCGGGCTGTAATTAGCGCTTG
GTTTAATGACGGCTCGTTTCTTTTCTGTGGCTGCGTGAAAGCCTTAAAGGGCTCCGGGAGG
GCCCTTTGTGCGGGGGGGAGCGGCTCGGGGGGTGCGTGCGTGTGTGTGTGCGTGGGGA
GCGCCGCGTGCGGCCCGCGCTGCCCGGCGGCTGTGAGCGCTGCGGGCGCGGCGCGGG
GCTTTGTGCGCTCCGCGTGTGCGCGAGGGGAGCGCGGCCGGGGGCGGTGCCCCGCGGT
GCGGGGGGGCTGCGAGGGGAACAAAGGCTGCGTGCGGGGTGTGTGCGTGGGGGGGTG
AGCAGGGGGTGTGGGCGCGGCGGTCGGGCTGTAACCCCCCCCTGCACCCCCCTCCCCG
AGTTGCTGAGCACGGCCCGGCTTCGGGTGCGGGGCTCCGTACGGGGCGTGGCGCGGGG
CTCGCCGTGCCGGGCGGGGGGTGGCGGCAGGTGGGGGTGCCGGGCGGGGCGGGGCC
GCCTCGGGCCGGGGAGGGCTCGGGGGAGGGGCGCGGCGGCCCCGGAGCGCCGGCGG
CTGTCGAGGCGCGGCGAGCCGCAGCCATTGCCTTTTATGGTAATCGTGCGAGAGGGCGC
AGGGACTTCCTTTGTCCCAAATCTGTGCGGAGCCGAAATCTGGGAGGCGCCGCCGCACCC
 
CCTCTAGCGGGCGCGGGGCGAAGCGGTGCGGCGCCGGCAGGAAGGAAATGGGCGGGG
AGGGCCTTCGTGCGTCGCCGCGCCGCCGTCCCCTTCTCCCTCTCCAGCCTCGGGGCTGT
CCGCGGGGGGACGGCTGCCTTCGGGGGGGACGGGGCAGGGCGGGGTTCGGCTTCTGG
CGTGTGACCGGCGGCTCTAGAGCCTCTGCTAACCATGTTCATGCCTTCTTCTTTTTCCTAC
AGAAGCTTCGAATTCTGCAGTCGACATGGCCCTGAGAGGCGTCTCCGTGCGGCTGCTGAG
CCGCGGACCCGGCCTGCACGTCCTTCGCACGTGGGTCTCGTCGGCGGCGCAGACCGAGA
AAGGCGGGAGAACACAGAGCCAACTGGCTAAGTCCTCGCGTCCCGAGTTTGACTGGCAG
GACCCGCTGGTGCTGGAGGAGCAGCTGACCACAGATGAGATCCTCATCAGGGACACCTTC
CGCACCTACTGCCAGGAGAGACTCATGCCTCGCATCCTGTTGGCCAATCGCAACGAAGTT
TTTCATCGGGAGATCATTTCGGAGATGGGGGAGTTGGGTGTGCTGGGCCCCACCATCAAA
GGATATGGCTGTGCTGGGGTTTCGTCTGTGGCCTATGGGCTCCTGGCCCGAGAGCTGGA
GCGGGTGGACAGTGGCTACAGGTCGGCGATGAGTGTCCAGTCCTCCCTCGTCATGCACC
CTATCTATGCCTATGGCAGCGAGGAACAGCGGCAGAAGTACCTGCCCCAGCTGGCCAAGG
GGGAGCTCCTGGGCTGCTTCGGGCTCACAGAGCCCAACAGCGGAAGTGACCCCAGCAGC
ATGGAGACCAGAGCCCACTACAACTCATCCAACAAGAGCTACACCCTCAATGGGACCAAG
ACCTGGATCACGAACTCGCCTATGGCCGATCTGTTTGTAGTGTGGGCTCGGTGTGAAGAT
GGCTGCATTCGGGGCTTCCTGCTGGAGAAGGGGATGCGGGGTCTCTCGGCCCCCAGGAT
CCAGGGCAAGTTCTCGCTGCGGGCCTCAGCCACAGGCATGATCATCATGGACGGTGTGG
AGGTGCCAGAGGAGAATGTGCTCCCTGGTGCATCCAGCCTGGGGGGTCCCTTCGGCTGC
CTGAACAACGCCCGGTACGGCATCGCGTGGGGCGTGCTTGGAGCTTCGGAGTTCTGCTT
GCACACAGCCCGGCAGTACGCCCTCGACAGGATGCAGTTTGGTGTCCCACTGGCCAGGA
ACCAGCTGATTCAGAAGAAGCTGGCAGACATGCTCACTGAGATTACCCTGGGCCTTCACG
CCTGCCTGCAGCTCGGCCGCTTGAAGGACCAGGACAAGGCTGCCCCCGAGATGGTTTCT
CTGCTGAAGAGGAATAACTGTGGGAAAGCCCTGGACATCGCCCGCCAGGCCCGAGACAT
GCTGGGGGGGAATGGGATTTCTGACGAGTATCACGTGATCCGGCACGCCATGAACCTGGA
GGCCGTGAACACCTACGAAGGTACACATGACATTCACGCCCTGATCCTTGGGAGAGCTAT
CACGGGAATCCAGGCGTTCACGGCCAGCAAGTTGTGAAGCTTAATCAACCTCTGGATTAC
AAAATTTGTGAAAGATTGACTGGTATTCTTAACTATGTTGCTCCTTTTACGCTATGTGGATAC
GCTGCTTTAATGCCTTTGTATCATGCTATTGCTTCCCGTATGGCTTTCATTTTCTCCTCCTTG
TATAAATCCTGGTTGCTGTCTCTTTATGAGGAGTTGTGGCCCGTTGTCAGGCAACGTGGCG
TGGTGTGCACTGTGTTTGCTGACGCAACCCCCACTGGTTGGGGCATTGCCACCACCTGTC
AGCTCCTTTCCGGGACTTTCGCTTTCCCCCTCCCTATTGCCACGGCGGAACTCATCGCCG
CCTGCCTTGCCCGCTGCTGGACAGGGGCTCGGCTGTTGGGCACTGACAATTCCGTGGTGT
TGTCGGGGAAATCATCGTCCTTTCCTTGGCTGCTCGCCTATGTTGCCACCTGGATTCTGCG
CGGGACGTCCTTCTGCTACGTCCCTTCGGCCCTCAATCCAGCGGACCTTCCTTCCCGCGG
CCTGCTGCCGGCTCTGCGGCCTCTTCCGCGTCTTCGCCTTCGCCCTCAGACGAGTCGGAT
CTCCCTTTGGGCCGCCTCCCCGCGGGTGGCATCCCTGTGACCCCTCCCCAGTGCCTCTCC
TGGCCCTGGAAGTTGCCACTCCAGTGCCCACCAGCCTTGTCCTAATAAAATTAAGTTGCAT
CATTTTGTCTGACTAGGTGTCCTTCTATAATATTATGGGGTGGAGGGGGGTGGTATGGAGC
AAGGGGCAAGTTGGGAAGACAACCTGTAGGGCCTGCGGGGTCTATTGGGAACCAAGCTG
GAGTGCAGTGGCACAATCTTGGCTCACTGCAATCTCCGCCTCCTGGGTTCAAGCGATTCTC
CTGCCTCAGCCTCCCGAGTTGTTGGGATTCCAGGCATGCATGACCAGGCTCAGCTAATTTT
TGTTTTTTTGGTAGAGACGGGGTTTCACCATATTGGCCAGGCTGGTCTCCAACTCCTAATC
TCAGGTGATCTACCCACCTTGGCCTCCCAAATTGCTGGGATTACAGGCGTGAACCACTGCT
CCCTTCCCTGTCCTT
**SEQ ID NO: 10** GCDH cDNA+ Seq cloning (**A**AGCTT Hind III; **GTCGAC** Sall; *GAATTC* ECORI) 
AAGCTTCGAATTCTGCAGTCGACATGGCCCTGAGAGGCGTCTCCGTGCGGCTGCTGAGCC
GCGGACCCGGCCTGCACGTCCTTCGCACGTGGGTCTCGTCGGCGGCGCAGACCGAGAAA
GGCGGGAGAACACAGAGCCAACTGGCTAAGTCCTCGCGTCCCGAGTTTGACTGGCAGGA
CCCGCTGGTGCTGGAGGAGCAGCTGACCACAGATGAGATCCTCATCAGGGACACCTTCCG
CACCTACTGCCAGGAGAGACTCATGCCTCGCATCCTGTTGGCCAATCGCAACGAAGTTTTT
CATCGGGAGATCATTTCGGAGATGGGGGAGTTGGGTGTGCTGGGCCCCACCATCAAAGG
ATATGGCTGTGCTGGGGTTTCGTCTGTGGCCTATGGGCTCCTGGCCCGAGAGCTGGAGC
GGGTGGACAGTGGCTACAGGTCGGCGATGAGTGTCCAGTCCTCCCTCGTCATGCACCCTA
TCTATGCCTATGGCAGCGAGGAACAGCGGCAGAAGTACCTGCCCCAGCTGGCCAAGGGG
GAGCTCCTGGGCTGCTTCGGGCTCACAGAGCCCAACAGCGGAAGTGACCCCAGCAGCAT
GGAGACCAGAGCCCACTACAACTCATCCAACAAGAGCTACACCCTCAATGGGACCAAGAC
CTGGATCACGAACTCGCCTATGGCCGATCTGTTTGTAGTGTGGGCTCGGTGTGAAGATGG
CTGCATTCGGGGCTTCCTGCTGGAGAAGGGGATGCGGGGTCTCTCGGCCCCCAGGATCC
AGGGCAAGTTCTCGCTGCGGGCCTCAGCCACAGGCATGATCATCATGGACGGTGTGGAG
GTGCCAGAGGAGAATGTGCTCCCTGGTGCATCCAGCCTGGGGGGTCCCTTCGGCTGCCT
GAACAACGCCCGGTACGGCATCGCGTGGGGCGTGCTTGGAGCTTCGGAGTTCTGCTTGC
ACACAGCCCGGCAGTACGCCCTCGACAGGATGCAGTTTGGTGTCCCACTGGCCAGGAAC
CAGCTGATTCAGAAGAAGCTGGCAGACATGCTCACTGAGATTACCCTGGGCCTTCACGCC
TGCCTGCAGCTCGGCCGCTTGAAGGACCAGGACAAGGCTGCCCCCGAGATGGTTTCTCTG
CTGAAGAGGAATAACTGTGGGAAAGCCCTGGACATCGCCCGCCAGGCCCGAGACATGCT
GGGGGGGAATGGGATTTCTGACGAGTATCACGTGATCCGGCACGCCATGAACCTGGAGG
CCGTGAACACCTACGAAGGTACACATGACATTCACGCCCTGATCCTTGGGAGAGCTATCAC
GGGAATCCAGGCGTTCACGGCCAGCAAGTTGTGAAGCTT
**SEQ ID NO: 11:** GCDH Protein 
MALRGVSVRLLSRGPGLHVLRTWVSSAAQTEKGGRTQSQLAKSSRPEFDWQDPLVLEEQLTT
DEILIRDTFRTYCQERLMPRILLANRNEVFHREIISEMGELGVLGPTIKGYGCAGVSSVAYGLLAR
ELERVDSGYRSAMSVQSSLVMHPIYAYGSEEQRQKYLPQLAKGELLGCFGLTEPNSGSDPSS
METRAHYNSSNKSYTLNGTKTWITNSPMADLFVVWARCEDGCIRGFLLEKGMRGLSAPRIQGK
FSLRASATGMIIMDGVEVPEENVLPGASSLGGPFGCLNNARYGIAWGVLGASEFCLHTARQYA
LDRMQFGVPLARNQLIQKKLADMLTEITLGLHACLQLGRLKDQDKAAPEMVSLLKRNNCGKAL
DIARQARDMLGGNGISDEYHVIRHAMNLEAVNTYEGTHDIHALILGRAITGIQAFTASKL
**SEQ ID NO: 12:** ITR 3' sequences

AGGAACCCCTAGTGATGGAGTTGGCCACTCCCTCTCTGCGCGCTCGCTCGCTCACTGAGG
CCGGGCGACCAAAGGTCGCCCGACGCCCGGGCTTTGCCCGGGCGGCCTCAGTGAGCGA
GCGAGCGCGCAGCTGCCTGCAGG

The invention is described below by the following examples, which must be considered as merely illustrative and in no case limiting of the scope of the present invention.

### EXAMPLES

### Materials and Methods

**Plasmid design and AAV vector generation.** To generate the pAAV-CAG-GCDH, the GCDH cDNA (Harvard Institute of Proteomics, HsCD00002837) was cloned into the pAAV-CA (Plasmid # 69616, addgene), under the control of the ubiquitous CAG promoter (chimeric promoter with the CMV enhancer and the chicken β-actin promoter). The AAV-GCDH vector was produced according to standard procedures at the Viral Vector Production Unit (UPV), Autonomous University of Barcelona.

**Western blot analysis.** Total protein extracts were obtained using lysis buffer (10 mM Tris-HCl [pH 6.8], 4% SDS, and 20% glycerol) containing 1% Complete Mini Protease Inhibitor (Roche). Lysates were boiled for 10 min at 98°C and centrifuged 5 min at 16,000 × *g*. Protein concentration was determined by BCA Protein Assay kit (ThermoFisher Scientific). Protein samples were resolved in 10% SDS-PAGE and transferred to nitrocellulose or PVDF membranes by standard methods. Membranes were blocked with TBS-Tween 10% milk (1 h at room temperature), immunoblotted with the corresponding antibody diluted in TBS-Tween 1% milk (Appendix Table S3), rinsed with TBS-Tween, and incubated with HPR-conjugated goat anti-rabbit or rabbit antimouse secondary antibodies (1/2000 in TBS-Tween 1% milk, 1h at room temperature). Antibody labelling was detected by ECL Amersham Prime Western Blotting Detection Reagent (GE Healthcare Life Sciences).

**GCDH in gel activity** GCDH activity was analyzed by blue-native polyacrilamide gel electrophoresis (BN-PAGE) using an adaptation of the original methodology described by Wittig et al., 2006. Briefly, liver and striatum from WT and GCDH KO mice were Dounce homogenized (20 strokes) in lysis buffer (225mM Manitol, 75mM Sucrose, 10mM Tris-HCl and 0.1mM EDTA) on ice. Tissue homogenates were centrifuged twice at 650g for 20 min at 4°C to remove cell debris and nuclei. Protein concentration was determined using a BCA assay. Mitochondria-enriched fractions were obtained from 1500µg of liver and 1000µg of striatum protein by centrifugation (10.0000g 10 min at 4°C). Mitochondria-enriched pellets were solubilized with 50mM Bis-Tris, 0.5M EDTA, 750mM aminocaproic acid in the presence of 2% N-Dodecyl-beta-Maltoside. Protein samples were loaded and separated in 4% to 20% polyacrylamide gradient gels in native-conditions. GCDH activity was visualized using a self-developed in gel activity assay by incubating the gel in a buffer containing Phosphate buffer 50mM, Glutaril CoA 200 µM), FAD 1,5 µM and NBT 2mg/ml at 37 °C O/N.

**Metabolite analysis.** Liver, striatum and brain homogenates were obtained as explained above. The glutarylcarnitine is extracted from, homogenated and plasma samples with an organic solvent containing deuterated acylcarnitines, including deuterated glutarylcarnitine used as internal standards (NeoBase 1 kit, Perkin Elmer). The extracted product is dried under and reconstituted in methanol/H₂O (75/25). In the case of neurological tissue samples the dried extracted product is derivatized with n-butanol/HCl 3N, dried under nitrogen and reconstituted in methanol/H₂O (75/25). Then the analysis is performed by ACQUITY UPLC system (I-Class, Waters, MA, USA)-XevoTQD in MRM mode, and with direct infusion using the mobile phase included in Neobase 1 kit. The quantification was done with Neolynx Software (Waters).

For glutaric acid and 3-OH-glutaric analysis, an aqueous solution containing the deuterium labeled internal standards GA-d4 and 3-OH-GA-d5, was added to the homogenized striatum samples. Compounds were extracted using an Oasis HLB 96-well Plate (60mg sorbent) and eluted using an acetonitrile/methanol (90/10) phase. To facilitate ionization, formic acid at 0,4% solution was added to the eluate. Chromatographic separation was done on an ACQUITY UPLC system (I-Class, Waters, MA, USA)-XevoTQS with an ACQUITY Premier BEH C18 Column (1.7 µm, 2.1 × 100 mm). The flow rate was set to 320 µL min⁻¹ using a binary mixture of solvent A (water with 0.1% formic acid) and solvent B (methanol with 0.1% formic acid). Values were quantified with Targetlynx Software (Walters) using a calibration curve and normalized for protein content.

**Animal procedures.** *Gcdh-*/*-* mice were purchased from the Mutant Mouse Resource and Research Center (MMRRC) strain ID 34368 and maintained in an SPF animal facility in a 12h dark-light cycle. Mice were fed ad libitum with a standard diet (ND) or a high lysine diet (HLD) 4,7% Lys when stated. Animals were placed in HLD at 3-week-old weanling or 5-week-old young adults. Mice were sacrificed at age of 1, 2 or 6 months. Gcdh-/- mice were injected in the temporal vein (neonatal) or tail vein (young adult) with 10¹¹ viral genomes of AAV9-GCDH. Neonatal injections were performed following the protocol described by Gombash Lampe et al.. Briefly, pups at P0, P1 or P2 were place on wet ice for 30-60 sec to anesthetize and placed under a stereoscope. The temporal vein was identified, the needle containing the virus was inserted into the vein and the viral content was released. Then, pups were coated with bedding to ensure acceptance by the mothers and placed back in the home page.

### Animal procedures met the guidelines of European Community Directive 86/609/EEC and were approved by the Local Ethical Committee

**Histological preparation and IHC analysis.** Mice were transcardially perfused with 1% PBS followed by 4% paraformaldehyde. Brains were removed and postfixed in 4% paraformaldehyde for 24 h at 4°C and embedded in paraffin. Serial 6 µm brain coronal sections were collected on glass slides. Sections were processed for antigen retrieval in sodium citrate buffer (10mM sodium citrate, 0.05% Tween-20, pH 6.0) at 95 °C for 30 min. Endogenous peroxidase was blocked with Dual Endogenous Enzyme Block (Dako) for 5 min. Sections were processed for antigen retrieval in sodium citrate buffer (10mM sodium citrate, 0.05% Tween-20, pH 6.0) at 95 °C for 30 min. Sections were treated with a blocking solution (PBS 1x, 10% FBS, 1% BSA, 0.3% triton X-100) for 1'5 h at room temperature.and incubated overnight at 4°C with primary antibodies ( Anti-Glial Fibrillary Acidic Protein (GFAP), G3893, Sigma-Aldrich; and anti-Myelin Basic Protein (MBP), clone SMI 94, Biolegend) diluted in PBS-T with 1% BSA. The reaction was developed using Dako EnVision + Dual Link System-HRP (DAB+) (Dako), and tissues were counterstained with Harris hematoxylin (Panreac). Stained sections were visualized with an Olympus IX51 inverted microscope and digitalized with a ScanScope slide scanner and analyzed with QuPath 0.3.0software.

**MRI studies and H¹ spectroscopy.** MRI experiments were performed in a cohort of 24 mice (12 female and 12 male) distributed in 4 experimental groups: WT n=6; KO without diet nor treatment n=6; KO with diet and no treatment n=6 and KO with diet and treatment n=6.
MRI acquisitions were performed on a 7.0T BioSpec 70/30 horizontal animal scanner (Bruker BioSpin, Ettlingen, Germany), equipped with an actively shielded gradient system (400 mT/m, 12 cm inner diameter). The receiver coil was a 1-channel phased-array surface coil for the mouse brain. The acquisition protocol included magnetic resonance spectroscopy (MRS) and diffusionweighted image (DWI).

**Statistical analysis.** Experimental data are represented by the mean ± SEM of at least three independent experiments. Statistical analysis was performed on GraphPad Prism v8.0.1 (GraphPad Software). Statistical differences were evaluated using a 2-tailed non-parametric Mann-Whitney test or a one-sample t-test. P < 0.05 was taken as the level of significance. Mice survival was analyzed by the Kaplan-Meier method and evaluated with a long-rank (Mantel-Cox) test.

### Intravascular delivery of AAV9-GCDH driven by the CAG promoter displays GCDH expression in brain and restores glutarylcarnitine accumulation

We constructed an AAV9 vector expressing the human GCDH gene under the control of the the chimeric CMV enhancer and chicken β-actin promoter (CAG) promoter (AAV-GCDH) (Figure 1). The CAG promoter was chosen for its ability to drive strong expression of the controlled genes. Since GCDH deficiency mostly leads to neuropathological alterations we selected the AAV serotype AAV9 for its widespread transduction of the CNS parenchyma and its ability to cross the blood-brain-barrier following intravascular delivery.

### Intravenous AAV-GCDH delivery to young adults prevents from diet-induced GA and 3-OH GA accumulation in the striatum five-months after therapy

To explore whether the AAV-GCDH vector was able to rescue the phenotype of *Gcdh* -/- mice when animals were fed with standard diet or undera high lysine diet. KO mice were injected in the tail vein with a single dose of 1×10¹¹ vg/mouse AAV-GCDH at 4-weeks old. One week later treated or control animals were placed under standard diet or a high lysine diet for one month or five months. Animals at 2-months old and 6-months old were killed and analyzed for GCDH expression, enzyme activity, and metabolites accumulation, GCDH was expressed in the liver of treated animals at similar levels to Wt mice at 2-months that decreased at 6-month of age (Figure 2A). Other peripheral tissues such as kidneys, also displayed GCDH expression, although at low levels (Figure 2B) Expression of GCDH was analyzed in the striatum, as the targeted brain region of GA I neuropathology, but very low levels of GCDH were detected at both one month and five months post-therapy (Figure 2C). Consistently, GCDH enzyme activity was detected at both ages in the liver, but no restoration was detected in the striatum (Figure 2D). The analysis of glutarylcarnitine (C5DC) accumulation revealed a statistically significant reduction of C5DC in the liver of treated mice maintained in standard diet that lasted until 6 months of age. Feeding the animals with HLD induced a remarkable increase in C5DC concentration that could not be compensated by the therapy (Figure 3A).

Although GCDH deficiency results in the accumulation of C5DC, GA and 3-OH GA, striatal neurotoxicity is attributed fundamentally to the activities of GA and 3-OH GA metabolites. Thus, to evaluate the impact of the AAV-GCDH treatment in the striatum we analyzed the content of the three metabolites. Animals treated with AAV-GCDH did not result in the correction of C5DC, GA and 3-OH GA metabolites when animals were fed in standard diet (Figure 3B, 3C, 3D). However, a reduction on GA and 3-OHGA after five months of high lysine diet were observed in the striatum of treated mice (Figure 3C, 3D). These results suggest that even low expression of GCDH is capable of preventing the strong induction of metabolites by the HLD.

### Intravascular neonatal AAV-GCDH administration improves the phenotype of Ggdh -/- mice exposed to high lysine diet

Since the GA-I pathology impacts individuals at early infancy the effects of the GCDH gene therapy would be desirable at early stages to prevent the development of encephalopathic crises. We therefore considered to evaluate the effects of AAV-GCDH therapy upon administration in neonatal mice.

*Gcdh -*/*-* KO mice were injected with 1×10¹¹ vg/mouse of AAV-GCDH vector into the temporal vein at neonatal P1-P2 stage. At 21 days after injection animals were placed in standard diet or under high lysine diet for 4 days or for five months. Animals were killed at one month of age or at 6 months (Figure 4A). GCDH expression in mice at one month after therapy showed high expression of the transgene in liver and striatum. At 6 months of age there was a remarkable reduction in the liver GCDH content, probably due to a dilution effect related to organ growth; however, GCDH expression was maintained in the striatum at similar levels of WT animals (Figure 4B). Glutary-coA dehydrogenase activity was detected both at one month and 6 months after therapy in the tissues analyzed (Figure 4C). Analysis of C5DC in the liver of treated animals showed metabolite correction at one month post-therapy but could not be sustained at 6 months post-treatment in HLD fed mice, probably related with the reduce expression of GCDH in the liver at this age (Figure 5A). Interestingly, AAV-GCDH therapy corrected the accumulation of C5DC and the neurotoxic metabolites GA and 3-OHGA in the striatum of HLD fed animals at both ages one and 6-months post-therapy (Figure 5B, 5C, 5D). Since GA-I pathology induced by the GA and 3 OH GA toxicity results in selective striatal degeneration we analyzed neuronal damage by measuring N-acetyl aspartate (NAA+NAAG)by ¹H NMR spectroscopy in the striatum. A statistically significant decrease in the NAA+NAAG content was observed in HLD *Gcdh* -/- mice that was rescued in treated animals (Figure 6A). Moreover, MRI analysis revealed bilateral striatal injury in HLD mice evidenced by reduced mean diffuse intensity that was less evident in mice one month after AAV-GCDH therapy (Figure 6B).

HLD exposure at weanlings has been associated with vacuolation, neuronal loss, myelin pathology and signs of gliosis (Zinanti 2006, Olivera-Bravo 2019). Histopathology of striatum of Gcdh -/- mice under HLD confirmed large irregular vacuolation that was almost absent in mice receiving AAV-GCDH. Chronic treatment with HLD for 6 months revealed severe vacuolation that was partially rescued upon treatment (Figure 7A). Immunohistochemistry against GFAP was abundant in HLD animals in line with diet-induced gliosis that was almost absent following therapy (Figure 7B). MBP staining revealed a progressive decrease in the number of myelinated areas in the striatum of HLD fed mice. AAV-GCDH treated mice displayed a myelination pattern similar to WT animals (Figure 7C).

Exposure to HLD at this early-stage of development has lethal consequences (Zinnanti, 2006). In our conditions close to 50% of Gcdh -/- mice succumb after one week of HLD diet and only 40% of animals survive the 6-months HLD. Of notice, all the animals receiving neonatal AAV-GCDH treatment survived and showed spontaneous activity as WT animals (Figure 8). These results suggest that neonatal AAV-GCDH gene therapy is effective in the diet-induced GA I preclinical model.

## Claims

1. A recombinant adeno-associated virus (AAV) genome comprising an expression cassette comprising:
(i) the nucleotide sequence of a chimeric promoter comprising the cytomegalovirus (CMV) enhancer, the chicken β-actin promoter and a chimeric intron,
(ii) the nucleotide sequence encoding for the human glutaryl-CoA dehydrogenase enzyme (GCDH) with an amino acid sequence that consists of SEQ ID NO: 11,
wherein (i) is operably linked to and regulates the expression of (ii).

2. The recombinant adeno-associated virus (AAV) genome according to claim 1, wherein the nucleotide sequence of the chimeric promoter consists of SEQ ID NO: 4.

3. The recombinant adeno-associated virus (AAV) genome according to any one of claims 1 or 2, wherein the expression cassette comprises SEQ ID NO: 9, or a sequence that has at least 85% sequence identity to the nucleotide sequence of SEQ ID NO: 9 and encodes for the human GCDH enzyme.

4. A recombinant adeno-associated virus (AAV) virion comprising: 1) an AAV capsid; and 2) the recombinant AAV genome of any one of claims 1 to 3; wherein the AAV capsid encapsulates said recombinant AAV genome.

5. The recombinant adeno-associated virus (AAV) virion according to claim 4, wherein the viral capsid is of serotype 9.

6. A cell comprising the recombinant adeno-associated virus (AAV) genome as defined in any of claims 1 to 3, or the recombinant AAV virion as defined in any of claims 4 or 5.

7. A pharmaceutical composition comprising the recombinant adeno-associated virus (AAV) genome as defined in any of claims 1 to 3, the AAV virion as defined in claims 4 or 5, or a substantially pure population of cells as defined in claim 6, and a pharmaceutically acceptable carrier or diluent.

8. The pharmaceutical composition as defined in claim 7, for use in the treatment of Glutaric aciduria type I.

9. The pharmaceutical composition for use according to claim 8, wherein the use comprises the intravascular administration of said pharmaceutical composition.

10. The pharmaceutical composition for use according to any of claims 8 or 9, wherein the use comprises the administration of the pharmaceutical composition in a human that is between 1-3 months old.
